# FASCICULE DE BREVET EUROPEEN

(11) **EP 2 440 195 B1**
(45) Date de publication et mention de la délivrance du brevet: **06.11.2019**
(21) Numéro de dépôt: 10737996.8
(22) Date de dépôt: 11.06.2010
(51) Int. Cl.: A61K 31/08, A61K 31/675, A61K 31/198, A61K 45/06, A61P 35/00

(54) **UTILISATION D'ALKYLGLYCEROLS POUR LA PREPARATION DE MEDICAMENTS**
VERWENDUNG VON ALKYLGLYCEROLEN ZUR HERSTELLUNG VON ARZNEIMITTELN
USE OF ALKYLGLYCEROLS FOR PREPARING DRUGS

(30) Priorité: 12.06.2009 FR 0953957
(43) Date de publication de la demande: 18.04.2012
(73) Titulaire: Université de Rennes I, 35065 Rennes Cedex (FR); Nutrialys Medical Nutrition SA, 35760 St Gregoire (FR)
(72) Inventeur: DENIAU, Anne-Laure, F-35000 Rennes (FR); MOULINOUX, Jacques-Philippe, F-35000 Rennes (FR); LEGRAND, Alain, Bernard, F-22270 Jugon-les-Lacs (FR)
(74) Mandataire: Grosset-Fournier, Chantal Catherine
(86) Numéro de dépôt international: PCT/FR2010/051171
(87) Numéro de publication internationale: WO 2010/142925

(56) Documents cités:
- WO-A1-95/00041
- WO-A1-98/32447
- ROBERT A CASERO AND LAURENCE J MARTON: "Targeting polyamine metabolism and function in cancer and other hyperproliferative diseases" NATURE REVIEWS. DRUG DISCOVERY, NATURE PUBLISHING GROUP, BASINGSTOKE, GB, vol. 6, 1 mai 2007 (2007-05-01), pages 373-390, XP007910866 ISSN: 1474-1784 [extrait le 2007-04-27]
- NIKOLAUS SEILER ET AL: "Endogenous and Exogenous Polyamines in Support of Tumor Growth" CANCER RESEARCH, AMERICAN ASSOCIATION FOR CANCER RESEARCH, BALTIMORE, MD., US, vol. 50, 15 août 1990 (1990-08-15), pages 5077-5083, XP007910864 ISSN: 0008-5472 cité dans la demande
- BROHULT A ET AL: "REDUCED MORTALITY IN CANCER PATIENTS AFTER ADMINISTRATION OF ALKOXYGLYCEROLS" ACTA OBSTETRICIA AND GYNECOLOGICA SCANDINAVICA, MUNKSGAARD, COPENHAGEN, DK, vol. 65, 1 janvier 1986 (1986-01-01), pages 779-785, XP000944112 ISSN: 0001-6349
- DATABASE MEDLINE [Online] US NATIONAL LIBRARY OF MEDICINE (NLM), BETHESDA, MD, US; septembre 1992 (1992-09), QUEMENER V ET AL: "Polyamine deprivation enhances antitumoral efficacy of chemotherapy." XP002560346 Database accession no. NLM1444206
- PEDRONO ET AL: "1-O-Alkylglycerols reduce the stimulating effects of bFGF on endothelial cell proliferation in vitro" CANCER LETTERS, NEW YORK, NY, US, vol. 251, no. 2, 3 mai 2007 (2007-05-03), pages 317-322, XP022059621 ISSN: 0304-3835
- JASNIS M A ET AL: "Polyamines prevent DFMO-mediated inhibition of angiogenesis" CANCER LETTERS, NEW YORK, NY, US, vol. 79, no. 1, 29 avril 1994 (1994-04-29) , pages 39-43, XP023162114 ISSN: 0304-3835 [extrait le 1994-04-29]

## Description

L'invention concerne l'utilisation d'alkylglycérols pour la préparation de médicaments en particulier destinés au traitement de pathologies tumorales.

Le développement des organismes supérieurs est caractérisé par un ensemble de divisions cellulaires régulées dans le temps et dans l'espace. Une perturbation dans la physiologie de la division cellulaire est presque toujours nuisible. Le cancer est une perturbation de ce type, une maladie qui peut résulter d'une série d'événements génétiques.

Les cellules cancéreuses sont définies par deux caractéristiques héritées, une croissance incontrôlée, et une invasion incontrôlée des tissus sains. Une cellule cancéreuse peut se diviser sans tenir compte des contraintes imposées à la division des cellules saines environnantes et conduire à la formation d'une tumeur. De plus, certaines cellules cancéreuses ont la capacité de migrer à partir de leur position initiale et d'envahir les tissus sains du patient.

Dans les formes les plus malignes et difficilement traitables de tumeurs, les contraintes de prolifération cellulaire normale sont complètement inopérantes, et les cellules tumorales s'échappent de leurs site d'origine pour métastaser dans d'autres parties de l'organisme. Plus une tumeur métastase, plus elle sera difficile à traiter et à éradiquer.

L'implication du métabolisme des polyamines dans la réplication cellulaire et donc dans les processus prolifératifs font de ce métabolisme l'une des cibles privilégiées des drogues antiprolifératives, en même temps que la source de nouveaux signaux circulants susceptibles de révéler l'existence d'un processus néoplasique au sein d'un organisme.

Les polyamines que l'on trouve non seulement à l'intérieur même des cellules mais également à l'état circulant dans les liquides biologiques de l'organisme, tels que le sang sont issues de trois sources principales :
- la prolifération cellulaire physiologique (croissance et/ou renouvellement des cellules constitutives de l'organisme) et tumorale,
- l'alimentation,
- les bactéries intestinales.

Différents travaux ont par ailleurs mis en évidence que, chez l'animal, l'administration conjointe :
- d'une alimentation dépourvue de polyamines,
- d'a-DFMO (inhibiteur de l'ornithine décarboxylase),
- d'un inhibiteur de la polyamine-oxydase (PAO) supprimant la rétro conversion oxydative de la spermidine et de la spermine en putrescine, et
- de néomycine et de métronidazole,
entraîne une inhibition quasi-totale de la progression tumorale du carcinome pulmonaire de Lewis 3LL (Seiler N. et al, Cancer Research, 1990, n°50, pp. 5077- 5083), du glioblastome humain U251 (Moulinoux J-Ph. et al, Anticancer Research, 1991, n°11, pp. 175-180), de l'adénocarcinome de la prostate Dunning MAT-LyLu (Moulinoux J-Ph. et al, Journal of Urology, 1991, n° 146, pp. 1408,1412) et du neuroblastome humain neuro 2a (Quemener et al, "Polyamines in the gastro-intestinal tract", Dowling R.H., Fôlsch I.R. et Lôser C Ed., Kluwer Académie -Publisher Boston, 1992, pp. 375-385) (Moulinoux J-Ph. et al WO95/00041).

Il a par ailleurs été également démontré chez l'animal que la déplétion en polyamines pouvait considérablement potentialiser les effets antiprolifératifs des drogues antitumorales conventionnelles (méthotrexate, cyclophosphamide, vindesine) tout en allongeant le temps de survie des animaux et pouvait permettre de réduire les quantités de drogues administrées tout en conservant le même effet antitumoral (Quemener V. et al, "Polyamine deprivation enhances antitumoral efficacy of chemotherapy", Anticancer Research n°12, 1992, pp. 1447-1454) (Cipolla B. et al., Urol Res. 1996;24(2):93-8).

Le métabolisme des polyamines comme cible privilégiée pour traiter cancers et maladies prolifératives est connu (Casero R. A. et Marton L. J. "Targeting polyamine metabolism and function in cancer and other hyperproliferative diseases", Nature Reviews. Drug Discovery, vol. 6, 2007, pages 373-390).

Les propriétés anti-angiogéniques de l'α-DFMO attribuables à la diminution du taux de polyamines sont connues (Jasnis M. A. et al., "Polyamines prevent DFMO-mediated inhibition of angiogenesis", Cancer Letters, vol. 79, no. 1, 1994, pages 39-43.).

Le document FR93/07586 (Moulinoux J-Ph. et al.) décrit une composition administrable à l'homme à titre d'aliment, de complément alimentaire et/ou de produit de nutrition thérapeutique, permettant de pallier l'inefficacité des traitements réalisés jusqu'ici chez l'homme par administration isolée d'a-DFMO et permettant d'obtenir l'inhibition de la prolifération des cellules tumorales, constituée d'un mélange nutritif pauvre en polyamines contenant moins de 50 picomoles/g de putrescine, de spermidine, de spermine et de cadavérine, comprenant, en pourcentage de poids sec par rapport au poids sec total : 10 % à 35 % de lipides, 8 % à 30 % de protéines, 35 % à 80 % de glucides, jusqu'à 10 % d'un mélange constitué de vitamines, de minéraux et d'électrolytes.

Les alkylglycérols sont des éthers de lipides. La forme diacétylée de certains d'entre eux est abondante dans l'huile de foie de requin.

Les alkylglycérols sont connus pour leur activité antitumorale et antimétastatique (Pedrono et al., Nutrition and Cancer, 2004, 48 (1) 64-69). L'utilisation de composés de type alkylglycérols dans le traitement du cancer du col de l'utérus (combiné avec de la radiothérapie) ou comme promoteurs d'anticorps est connu (Brohult A. et al. "Reduced mortality in cancer patients after administration of alkoxyglycerols", Acta Obstetricia and Gynecologica Scandinavica, vol. 65, 1986, pages 779-785).

La combinaison entre alkylglycérols et un agent chimiothérapeutique, est décrite notamment dans le cadre du traitement de tumeurs malignes, et ce quel que soit l'agent de chimiothérapie (Firshein WO 98/32 447).

L'un des buts de l'invention est de proposer un médicament qui contient au moins un inhibiteur de la biosynthèse des polyamines, et qui est plus efficace que l'action dudit inhibiteur de la biosynthèse des polyamines seul, dans le cadre du traitement de pathologies, associées à un dysfonctionnement de l'angiogénèse, et / ou de pathologies tumorales.

L'un des buts de l'invention est de proposer un médicament qui contient au moins un alkylglycérol, et qui est plus efficace que l'action dudit alkylglycérol seul, dans le cadre du traitement de pathologies, associées à un dysfonctionnement de l'angiogénèse, et / ou de pathologies tumorale.

L'un des buts de l'invention est de proposer une combinaison pharmaceutique de composants présentant une synergie par rapport à la somme des effets des composants pris individuellement.

L'un des buts de l'invention est de proposer une composition alimentaire comprenant des composants présentant une synergie par rapport à la somme des effets des composants pris individuellement et susceptible d'être associée à un traitement antitumoral ou de pathologie liées au dysfonctionnement de l'angiogénèse.

La présente invention concerne l'utilisation d'un produit contenant :
- au moins un alkylglycérol,
- au moins un inhibiteur de la biosynthèse des polyamines, ledit inhibiteur étant un inhibiteur de l'omithine décarboxylase,
- un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et
- au moins un agent antitumoral, comme préparation de combinaison pour une utilisation simultanée, séparée ou étalée dans le temps, pour la fabrication d'un médicament destiné au traitement de pathologies associées à un dysfonctionnement de l'angiogénèse, et / ou de pathologies tumorales,
la quantité totale de polyamines biologiquement «actives» par jour ingérées par l'homme ou l'animal, auquel le susdit médicament est administré, ne dépassant pas 30 nano moles par kcal de produit ingéré, notamment 25 nanomoles par kcal de produit ingéré, notamment 23 nanomoles par kcal de produit ingéré, notamment 20 nanomoles par kcal de produit ingéré.

L'invention est telle que définie dans les revendications 1-13.

On a trouvé, de façon surprenante, l'existence d'un effet synergique, dans le traitement des pathologies associées à un dysfonctionnement de l'angiogénèse, et / ou de pathologies tumorales, reposant sur l'utilisation d'au moins un alkylglycérol, d'au moins un inhibiteur de la biosynthèse des polyamines biologiquement actives, et éventuellement d'un agent antitumoral, dans le cadre d'un régime pauvre en polyamines.

Le patient ou l'animal auquel est administré le produit selon la présente invention ne doit pas absorber une quantité de polyamines biologiquement actives supérieure à 9 mg / jour. En effet, il a été observé que les polyamines biologiquement actives favorisent le développement tumoral et l'angiogénèse. La prise d'une quantité de polyamines supérieure à cette valeur annulerait la synergie observée lors de l'utilisation du produit selon la présente invention.

Par « excipient appauvri en polyamines actives », on désigne un excipient contenant peu de polyamines, c'est-à-dire un excipient dans lequel le taux total de polyamines actives est 100 fois inférieur, notamment 500 fois inférieur, notamment 1000 fois inférieur à la quantité de polyamines actives présentes dans un régime alimentaire normal.

Un régime alimentaire normal fournissant un apport quotidien de 1950 kcal, pour un adulte de 70 kg, peut contenir, en fonction des aliments consommés, de 80 000 à 700 000 nmol de polyamines biologiquement actives par jour, soit de 40 à 360 nmol de polyamines biologiquement actives par kcal par jour.

Une alimentation standard fournit un apport de 118 nmol de polyamines biologiquement actives par kcal par jour.

Pour pouvoir convertir des quantités exprimées en nanomoles de polyamines par kilocalories en grammes de polyamines par kilocalories, il est nécessaire de considérer une masse moléculaire moyenne pour l'ensemble des polyamines. Cette masse moléculaire des polyamines est une approximation nécessaire pour réaliser ce calcul, elle est estimée à 145,24 g/mol.

Par « polyamines biologiquement actives », on désigne les polyamines qui ont un effet sur :
- la stabilisation, la condensation et la conformation de l'ADN (Thomas & Thomas (2001) "Polyamines in cell growth and cell death : molecular mechanism and therapeutic applications." CMLS, Cell Mol Life Sci 58 : 244-258),
- la transcription de l'ARN,
- la croissance et la prolifération cellulaire en intervenant directement sur le cycle cellulaire des cellules (Thomas & Thomas 2001),
- la régulation de la réponse immune (Soulet D & Rivest S (2003) "Polyamines play a critical role in the control of the innate immune response in the mouse central nervous system." The Journal of Cell Biology vol. 162 ; n°2 July 21 : 257-268),
- la modulation du fonctionnement des récepteurs N-méthyl-D-asparthate (NMDA) et sont impliquées dans les processus de neurodégénéréscence (Soulet & Rivest 2003).

Les polyamines biologiquement « actives » sont les polyamines (putrescine, cadavérine, spermine et spermidine seront citées précisément) présentes dans le mélange sans être chélatées, protégées, ou masquées.

Les polyamines biologiquement « actives » peuvent être identifiées notamment selon les méthodes suivantes :

### 1) En culture

Une polyamine biologiquement « active » ou l'un de ses dérivés doit pouvoir participer au métabolisme cellulaire physiologique des polyamines, voire être capable d'interférer avec ce dernier ou encore de le dysréguler.
a. Une polyamine biologiquement « active » doit donc pouvoir s'associer voire être reconnue par le ou les systèmes de transport visant à l'internaliser dans une cellule vivante. L'adjonction d'une polyamine biologiquement « active » radiomarquée au milieu de culture permet de constater son internalisation.
b. Une polyamine biologiquement « active » doit pouvoir supprimer l'inhibition de la prolifération cellulaire provoquée par l'inhibition de l'anabolisme endogène des polyamines (DFMO).
c. Une polyamine biologiquement « active », y compris de synthèse, en dysrégulant le métabolisme naturel des polyamines doit pouvoir moduler le niveau de la prolifération cellulaire.

### 2) In vivo :

Chez l'animal porteur de tumeur greffée, l'apport exogène (tractus gastro-intestinal) de polyamines doit supprimer les effets anticancéreux bénéfiques provoqués par la carence en polyamines induite par la diminution des sources endogènes et exogènes de polyamines, couplée ou non à des médicaments anticancéreux.

Par « alkylglycérol », on désigne une molécule comprenant un groupement glycérol comme élément de ramification, et au moins une chaîne carbonée liée par une fonction éther au groupement glycérol.

Il est important de noter que les alkylglycérols de la combinaison selon la présente invention sont distincts des alkylglycérols pouvant être présents de façon endogène dans l'organisme du patient ou de l'animal, traité avec le médicament selon la présente invention.

Les alkylglycérols administrés selon la présente invention sont exogènes, par opposition aux alkylglycérols endogènes présents dans l'organisme d'un patient ou d'un animal. Les alkylglycérols endogènes se trouvent dans l'organisme sous forme diacétylée.

Par « inhibiteur de la biosynthèse des polyamines », on désigne une molécule capable de bloquer, totalement ou partiellement, directement ou indirectement, au moins l'une des enzymes intervenant dans la synthèse des polyamines dans l'organisme humain, ou animal. L'ornithine décarboxylase (EC 4.1.1.17) est une enzyme cible pour les composés inhibiteurs de la biosynthèse des polyamines. Le rôle de l'inhibiteur de la biosynthèse des polyamines est d'arrêter ou de réduire significativement la production endogène de polyamines dans l'organisme traité avec le produit selon la présente invention. La mise en œuvre conjointe d'un inhibiteur de la biosynthèse des polyamines et d'un apport alimentaire pauvre en polyamines permet de réduire la quantité de polyamines biodisponibles dans l'organisme.

Par « dysfonctionnement de l'angiogénèse », on désigne un trouble des processus d'apparition et / ou de croissance de nouveaux vaisseaux sanguins, ces processus se nommant également néo vascularisation.

Par « pathologies tumorales », on désigne une pathologie liée à la néoformation de tissus corporels suite au dérèglement de la croissance cellulaire. Ces pathologies peuvent toucher tous les types de tissus cellulaires. Elles peuvent entrainer des troubles dans le fonctionnement des organes, nuire au fonctionnement de l'organisme et éventuellement entrainer la mort. Les cancers sont des pathologies tumorales.

La quantité de médicament administré au patient ou à l'animal, est déterminée par rapport aux besoins quotidiens du patient ou de l'animal en nutriments. Les besoins en nutriments sont déterminés par rapport à la masse corporelle du patient ou de l'animal absorbant la combinaison.

Les apports en nutriments nécessaires au patient ou à l'animal, notamment en calories, c'est-à-dire en énergie nécessaire au fonctionnement de l'organisme, sont connus de l'homme du métier.

Par « nutriments », on désigne tous les composés nécessaires au maintien des fonctions vitales d'un organisme, tels que notamment les glucides, lipides, protéines, sels minéraux et vitamines.

La présente invention implique un contrôle des apports en polyamines biologiquement actives. Selon l'invention, ces apports peuvent provenir du médicament ou de la diète totale, c'est-à-dire l'alimentation globale du patient ou de l'animal. La diète totale inclut le médicament.

L'apport en polyamines biologiquement actives de la diète totale inclut l'apport lié à l'absorption du médicament.

Afin de contrôler correctement les apports en polyamines biologiquement actives, il est nécessaire de connaitre les teneurs en polyamines du médicament, et de l'ensemble des aliments constituant la diète totale.

Le régime alimentaire pauvre en polyamines biologiquement «actives » est obtenu en contrôlant la teneur en polyamines de l'alimentation du patient ou de l'animal.

En résumé, le contrôle des apports en polyamines biologiquement actives comprend deux niveaux distincts : la diète totale et le médicament. La présente invention implique un contrôle de ces deux niveaux.

L'alkylglycérol, l'inhibiteur de la biosynthèse des polyamines, et l'excipient appauvri en polyamines peuvent être administrés conjointement ou séparément, lors des repas ou répartis dans la journée.

Notamment lorsque l'alkylglycérol et l'inhibiteur sont administrés séparément, le médicament de l'invention est un kit dans lequel d'une part l'alkylglycérol est sous une forme galénique appropriée, et d'autre part l'inhibiteur de la biosynthèse des polyamines est sous une forme galénique appropriée, le tout étant administré avec un régime alimentaire pauvre en polyamines biologiquement «actives».

Ce mode de traitement offre plusieurs avantages, notamment la possibilité d'adapter à chaque individu traité :
le degré de pauvreté de l'alimentation en polyamines,
la quantité d'alkylglycérol administrée, et
la quantité d'inhibiteurs de la biosynthèse des polyamines administrée.

Selon un mode de réalisation de la présente invention, le médicament présenté sous forme de kit comprend au moins un alkylglycérol sous une forme galénique appropriée, au moins un inhibiteur de la biosynthèse des polyamines sous une forme galénique appropriée, et au moins un agent antitumoral sous une forme galénique appropriée, le tout administré avec un régime alimentaire pauvre en polyamines biologiquement «actives».

Comme décrit précédemment l'utilisation d'un tel kit permet d'adapter les dosages en agent anti-tumoral à chaque individu traité, indépendamment des dosages en alkylglycérol, en inhibiteur de la biosynthèse des polyamines, ou du degré de pauvreté de son régime alimentaire en polyamines biologiquement actives.

Ce kit permet également d'utiliser des agents antitumoraux qui risqueraient de se dégrader lors de la préparation de la combinaison objet de la présente invention. Ainsi, une plus grande variété d'agents antitumoraux peut être utilisée dans le cadre de la présente invention.

Ce kit permet aussi de mieux contrôler la fréquence dans le temps de l'administration des agents antitumoraux. Ainsi le patient, ou l'animal, peut subir les administrations d'agent antitumoral à un rythme optimal en fonction de sa pathologie et de son état de santé.

Par « forme galénique appropriée » de l'alkylglycérol, de l'inhibiteur de la biosynthèse des polyamines ou de l'agent antitumoral, on désigne un conditionnement permettant une utilisation aisée. A titre d'exemple on peut citer les poudres, hydrosolubles ou non, comprimés, pastilles, gélules, gouttes, nébulisateurs, patchs, ampoules pour injections

Selon un mode de réalisation avantageux, la présente invention concerne l'utilisation d'un produit contenant :
- au moins un alkylglycérol,
- au moins un inhibiteur de la biosynthèse des polyamines, ledit inhibiteur étant un inhibiteur de l'omithine décarboxylase, et
- un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine,
comme préparation de combinaison pour une utilisation simultanée, séparée ou étalée dans le temps, pour la fabrication d'un médicament destiné au traitement de pathologies associées à un dysfonctionnement de l'angiogénèse, et / ou de pathologies tumorales,
la quantité totale de polyamines biologiquement «actives» par jour ingérées par l'homme ou l'animal, auquel le susdit médicament est administré, ne dépassant pas 30 nano moles par kcal de produit ingéré, notamment 25 nanomoles par kcal de produit ingéré, notamment 23 nanomoles par kcal de produit ingéré, notamment 20 nanomoles par kcal de produit ingéré.

Dans un mode de réalisation particulier de l'invention, deux des composants du produit de combinaison sont déjà sous forme de pré-combinaison. Ce mode de réalisation permet de contourner avantageusement les problèmes liés à la stabilité des composants, ce mode de réalisation permet également de faciliter la prise du traitement par le patient ou l'animal en réduisant le nombre de composants à absorber.

Par « stabilité des composants » on désigne la stabilité chimique des composants en fonction du milieu dans lequel ils se trouvent. Par exemple, certaines molécules peuvent se dégrader en milieux aqueux, ou se dégrader en présence d'oxygène, ou d'un pH particulier. Regrouper les molécules présentant les mêmes contraintes de conservation permet de réduire les risques d'absorption d'un composant dégradé lors du traitement.

Selon un mode de réalisation avantageux, la présente invention concerne l'utilisation d'un produit contenant :
- une combinaison d'un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et d'au moins un alkylglycérol, et
- au moins un inhibiteur de la biosynthèse des polyamines, ledit inhibiteur étant un inhibiteur de l'omithine décarboxylase,
comme préparation de combinaison pour une utilisation simultanée, séparée ou étalée dans le temps, pour la fabrication d'un médicament destiné au traitement de pathologies associées à un dysfonctionnement de l'angiogénèse, et / ou de pathologies tumorales,
la quantité totale de polyamines biologiquement «actives» par jour ingérées par l'homme ou l'animal, auquel le susdit médicament est administré, ne dépassant pas 30 nano moles par kcal de produit ingéré, notamment 25 nanomoles par kcal de produit ingéré, notamment 23 nanomoles par kcal de produit ingéré, notamment 20 nanomoles par kcal de produit ingéré.

Selon un mode de réalisation avantageux, la présente invention concerne l'utilisation d'un produit contenant :
- une combinaison d'un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et d'au moins un inhibiteur de la biosynthèse des polyamines, ledit inhibiteur étant un inhibiteur de l'omithine décarboxylase, et
- au moins un alkylglycérol,
comme préparation de combinaison pour une utilisation simultanée, séparée ou étalée dans le temps, pour la fabrication d'un médicament destiné au traitement de pathologies associées à un dysfonctionnement de l'angiogénèse, et / ou de pathologies tumorales,
la quantité totale de polyamines biologiquement «actives» par jour ingérées par l'homme ou l'animal, auquel le susdit médicament est administré, ne dépassant pas 30 nano moles par kcal de produit ingéré, notamment 25 nanomoles par kcal de produit ingéré, notamment 23 nanomoles par kcal de produit ingéré, notamment 20 nanomoles par kcal de produit ingéré.

Selon un mode de réalisation avantageux, la présente invention concerne l'utilisation d'un produit contenant :
- une combinaison d'au moins un alkylglycérol et d'au moins un inhibiteur de la biosynthèse des polyamines, ledit inhibiteur étant un inhibiteur de l'omithine décarboxylase, et
- un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine,
comme préparation de combinaison pour une utilisation simultanée, séparée ou étalée dans le temps, pour la fabrication d'un médicament destiné au traitement de pathologies associées à un dysfonctionnement de l'angiogénèse, et / ou de pathologies tumorales,
la quantité totale de polyamines biologiquement «actives» par jour ingérées par l'homme ou l'animal, auquel le susdit médicament est administré, ne dépassant pas 30 nano moles par kcal de produit ingéré, notamment 25 nanomoles par kcal de produit ingéré, notamment 23 nanomoles par kcal de produit ingéré, notamment 20 nanomoles par kcal de produit ingéré.

Selon un mode de réalisation avantageux, la présente invention concerne l'utilisation d'un produit contenant :
- au moins un alkylglycérol,
- au moins un inhibiteur de la biosynthèse des polyamines, ledit inhibiteur étant un inhibiteur de l'omithine décarboxylase,
- un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et
- au moins un agent antitumoral,
comme préparation de combinaison pour une utilisation simultanée, séparée ou étalée dans le temps, pour la fabrication d'un médicament destiné au traitement de pathologies associées à un dysfonctionnement de l'angiogénèse, et / ou de pathologies tumorales,
la quantité totale de polyamines biologiquement «actives» par jour ingérées par l'homme ou l'animal, auquel le susdit médicament est administré, ne dépassant pas 30 nano moles par kcal de produit ingéré, notamment 25 nanomoles par kcal de produit ingéré, notamment 23 nanomoles par kcal de produit ingéré, notamment 20 nanomoles par kcal de produit ingéré.

Selon un mode de réalisation avantageux, la présente invention concerne l'utilisation d'un produit contenant :
- une combinaison d'un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et
   d'au moins un alkylglycérol,
- au moins un inhibiteur de la bio synthèse des polyamines, ledit inhibiteur étant un inhibiteur de l'omithine décarboxylase, et
- au moins un agent antitumoral,
comme préparation de combinaison pour une utilisation simultanée, séparée ou étalée dans le temps, pour la fabrication d'un médicament destiné au traitement de pathologies associées à un dysfonctionnement de l'angiogénèse, et / ou de pathologies tumorales,
la quantité totale de polyamines biologiquement «actives» par jour ingérées par l'homme ou l'animal, auquel le susdit médicament est administré, ne dépassant pas 30 nano moles par kcal de produit ingéré, notamment 25 nanomoles par kcal de produit ingéré, notamment 23 nanomoles par kcal de produit ingéré, notamment 20 nanomoles par kcal de produit ingéré.

Selon un mode de réalisation avantageux, la présente invention concerne l'utilisation d'un produit contenant :
- une combinaison d'au moins un inhibiteur de la biosynthèse des polyamines, ledit inhibiteur étant un inhibiteur de l'omithine décarboxylase, et d'au moins un agent antitumoral,
- au moins un alkylglycérol, et
- un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine,
comme préparation de combinaison pour une utilisation simultanée, séparée ou étalée dans le temps, pour la fabrication d'un médicament destiné au traitement de pathologies tumorales, la quantité totale de polyamines biologiquement «actives» par jour ingérées par l'homme ou l'animal, auquel le susdit médicament est administré, ne dépassant pas 30 nano moles par kcal de produit ingéré, notamment 25 nanomoles par kcal de produit ingéré, notamment 23 nanomoles par kcal de produit ingéré, notamment 20 nanomoles par kcal de produit ingéré.

Selon un mode de réalisation avantageux, la présente invention concerne l'utilisation d'un produit contenant :
- une combinaison d'un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et d'au moins un inhibiteur de la biosynthèse des polyamines,
- au moins un alkylglycérol, et
- au moins un agent antitumoral,
comme préparation de combinaison pour une utilisation simultanée, séparée ou étalée dans le temps, pour la fabrication d'un médicament destiné au traitement de pathologies tumorales,
la quantité totale de polyamines biologiquement «actives» par jour ingérées par l'homme ou l'animal, auquel le susdit médicament est administré, ne dépassant pas 30 nano moles par kcal de produit ingéré, notamment 25 nanomoles par kcal de produit ingéré, notamment 23 nanomoles par kcal de produit ingéré, notamment 20 nanomoles par kcal de produit ingéré.

Selon un mode de réalisation avantageux, la présente invention l'utilisation d'un produit contenant :
- une combinaison d'au moins un alkylglycérol et d'au moins un agent antitumoral,
- au moins un inhibiteur de la biosynthèse des polyamines, ledit inhibiteur étant un inhibiteur de l'omithine décarboxylase, et
- un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine,
comme préparation de combinaison pour une utilisation simultanée, séparée ou étalée dans le temps, pour la fabrication d'un médicament destiné au traitement de pathologies associées à un dysfonctionnement de l'angiogénèse, et / ou de pathologies tumorales,
la quantité totale de polyamines biologiquement «actives» par jour ingérées par l'homme ou l'animal, auquel le susdit médicament est administré, ne dépassant pas 30 nano moles par kcal de produit ingéré, notamment 25 nanomoles par kcal de produit ingéré, notamment 23 nanomoles par kcal de produit ingéré, notamment 20 nanomoles par kcal de produit ingéré.

Selon un mode de réalisation avantageux, la présente invention concerne l'utilisation d'un produit contenant :
- une combinaison d'un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et d'au moins un agent antitumoral,
- au moins un alkylglycérol, et
- au moins un inhibiteur de la biosynthèse des polyamines, ledit inhibiteur étant un inhibiteur de l'omithine décarboxylase,
comme préparation de combinaison pour une utilisation simultanée, séparée ou étalée dans le temps, pour la fabrication d'un médicament destiné au traitement de pathologies tumorales, la quantité totale de polyamines biologiquement «actives» par jour ingérées par l'homme ou l'animal, auquel le susdit médicament est administré, ne dépassant pas 30 nano moles par kcal de produit ingéré, notamment 25 nanomoles par kcal de produit ingéré, notamment 23 nanomoles par kcal de produit ingéré, notamment 20 nanomoles par kcal de produit ingéré.

Selon un mode de réalisation avantageux, la présente invention concerne l'utilisation d'un produit contenant :
- une combinaison d'au moins un alkylglycérol et d'au moins un inhibiteur de la biosynthèse des polyamines, ledit inhibiteur étant un inhibiteur de l'omithine décarboxylase,
- d'au moins un agent antitumoral, et
- un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine,
comme préparation de combinaison pour une utilisation simultanée, séparée ou étalée dans le temps, pour la fabrication d'un médicament destiné au traitement de pathologies tumorales, la quantité totale de polyamines biologiquement «actives» par jour ingérées par l'homme ou l'animal, auquel le susdit médicament est administré, ne dépassant pas 30 nano moles par kcal de produit ingéré, notamment 25 nanomoles par kcal de produit ingéré, notamment 23 nanomoles par kcal de produit ingéré, notamment 20 nanomoles par kcal de produit ingéré.

Dans un mode de réalisation particulier de l'invention, les composants du produit de combinaison sont déjà, deux à deux, sous forme de pré-combinaison. Ce mode de réalisation permet de contourner avantageusement les problèmes liés à la stabilité des composants, ce mode de réalisation permet également de faciliter la prise du traitement par le patient ou l'animal en réduisant le nombre de composants à absorber.

Selon un mode de réalisation avantageux, la présente invention concerne l'utilisation d'un produit contenant :
- une combinaison d'un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et d'au moins un alkylglycérol, et
- une combinaison d'au moins un inhibiteur de la biosynthèse des polyamines, ledit inhibiteur étant un inhibiteur de l'omithine décarboxylase, et d'au moins un agent antitumoral,
comme préparation de combinaison pour une utilisation simultanée, séparée ou étalée dans le temps, pour la fabrication d'un médicament destiné au traitement de pathologies tumorales, la quantité totale de polyamines biologiquement «actives» par jour ingérées par l'homme ou l'animal, auquel le susdit médicament est administré, ne dépassant pas 30 nano moles par kcal de produit ingéré, notamment 25 nanomoles par kcal de produit ingéré, notamment 23 nanomoles par kcal de produit ingéré, notamment 20 nanomoles par kcal de produit ingéré.

Selon un mode de réalisation avantageux, la présente invention concerne l'utilisation d'un produit contenant :
- une combinaison d'un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et d'au moins un inhibiteur de la biosynthèse des polyamines, ledit inhibiteur étant un inhibiteur de l'omithine décarboxylase,
- une combinaison d'au moins un alkylglycérol, et d'au moins un agent antitumoral, comme préparation de combinaison pour une utilisation simultanée, séparée ou étalée dans le temps, pour la fabrication d'un médicament destiné au traitement de pathologies tumorales,
la quantité totale de polyamines biologiquement «actives» par jour ingérées par l'homme ou l'animal, auquel le susdit médicament est administré, ne dépassant pas 30 nano moles par kcal de produit ingéré, notamment 25 nanomoles par kcal de produit ingéré, notamment 23 nanomoles par kcal de produit ingéré, notamment 20 nanomoles par kcal de produit ingéré.

Selon un mode de réalisation avantageux, la présente invention concerne l'utilisation d'un produit contenant :
- une combinaison d'un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et d'au moins un agent antitumoral, et
- une combinaison d'au moins un alkylglycérol et d'au moins un inhibiteur de la biosynthèse des polyamines, ledit inhibiteur étant un inhibiteur de l'omithine décarboxylase,
comme préparation de combinaison pour une utilisation simultanée, séparée ou étalée dans le temps, pour la fabrication d'un médicament destiné au traitement de pathologies tumorales, la quantité totale de polyamines biologiquement «actives» par jour ingérées par l'homme ou l'animal, auquel le susdit médicament est administré, ne dépassant pas 30 nano moles par kcal de produit ingéré, notamment 25 nanomoles par kcal de produit ingéré, notamment 23 nanomoles par kcal de produit ingéré, notamment 20 nanomoles par kcal de produit ingéré.

Dans un mode de réalisation particulier de l'invention, trois des composants du produit de combinaison sont déjà sous forme de pré-combinaison. Ce mode de réalisation permet de contourner avantageusement les problèmes liés à la stabilité des composants, ce mode de réalisation permet également de faciliter la prise du traitement par le patient ou l'animal en réduisant le nombre de composants à absorber.

Selon un mode de réalisation avantageux, la présente invention concerne l'utilisation d'un produit contenant :
- une combinaison d'un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, d'au moins un alkylglycérol et d'au moins un inhibiteur de la biosynthèse des polyamines, ledit inhibiteur étant un inhibiteur de l'omithine décarboxylase, et
- au moins un agent antitumoral
comme préparation de combinaison pour une utilisation simultanée, séparée ou étalée dans le temps, pour la fabrication d'un médicament destiné au traitement de pathologies tumorales, la quantité totale de polyamines biologiquement «actives» par jour ingérées par l'homme ou l'animal, auquel le susdit médicament est administré, ne dépassant pas 30 nano moles par kcal de produit ingéré, notamment 25 nanomoles par kcal de produit ingéré, notamment 23 nanomoles par kcal de produit ingéré, notamment 20 nanomoles par kcal de produit ingéré.

Selon un mode de réalisation avantageux, la présente invention concerne l'utilisation d'un produit contenant :
- une combinaison d'au moins un alkylglycérol, d'au moins un agent antitumoral, et d'au moins un inhibiteur de la biosynthèse des polyamines, ledit inhibiteur étant un inhibiteur de l'omithine décarboxylase, et
- un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine,
comme préparation de combinaison pour une utilisation simultanée, séparée ou étalée dans le temps, pour la fabrication d'un médicament destiné au traitement de pathologies tumorales, la quantité totale de polyamines biologiquement «actives» par jour ingérées par l'homme ou l'animal, auquel le susdit médicament est administré, ne dépassant pas 30 nano moles par kcal de produit ingéré, notamment 25 nanomoles par kcal de produit ingéré, notamment 23 nanomoles par kcal de produit ingéré, notamment 20 nanomoles par kcal de produit ingéré.

Selon un mode de réalisation avantageux, la présente invention concerne l'utilisation d'un produit contenant :
- une combinaison d'un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, d'au moins un agent antitumoral, et d'au moins un inhibiteur de la biosynthèse des polyamines, ledit inhibiteur étant un inhibiteur de l'omithine décarboxylase, et
- d'au moins un alkylglycérol,
comme préparation de combinaison pour une utilisation simultanée, séparée ou étalée dans le temps, pour la fabrication d'un médicament destiné au traitement de pathologies tumorales, la quantité totale de polyamines biologiquement «actives» par jour ingérées par l'homme ou l'animal, auquel le susdit médicament est administré, ne dépassant pas 30 nano moles par kcal de produit ingéré, notamment 25 nanomoles par kcal de produit ingéré, notamment 23 nanomoles par kcal de produit ingéré, notamment 20 nanomoles par kcal de produit ingéré.

Selon un mode de réalisation avantageux, la présente invention concerne l'utilisation d'un produit contenant :
- une combinaison d'un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, d'au moins un agent antitumoral, et d'au moins un alkylglycérol, et
- d'au moins un inhibiteur de la biosynthèse des polyamines, ledit inhibiteur étant un inhibiteur de l'omithine décarboxylase,
comme préparation de combinaison pour une utilisation simultanée, séparée ou étalée dans le temps, pour la fabrication d'un médicament destiné au traitement de pathologies tumorales, la quantité totale de polyamines biologiquement «actives» par jour ingérées par l'homme ou l'animal, auquel le susdit médicament est administré, ne dépassant pas 30 nano moles par kcal de produit ingéré, notamment 25 nanomoles par kcal de produit ingéré, notamment 23 nanomoles par kcal de produit ingéré, notamment 20 nanomoles par kcal de produit ingéré.

Selon un mode de réalisation avantageux, la présente invention concerne l'utilisation d'une composition contenant au moins un alkylglycérol, au moins un inhibiteur de la biosynthèse des polyamines, ledit inhibiteur étant un inhibiteur de l'omithine décarboxylase, un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et ne contenant pas d'agent antitumoral, pour la préparation d'un médicament, pour l'homme ou l'animal, destiné au traitement de pathologies tumorales,
la quantité totale de polyamines biologiquement «actives» par jour ingérées par l'homme ou l'animal, auquel le susdit médicament est administré, ne dépassant pas 30 nano moles par kcal de produit ingéré, notamment 25 nanomoles par kcal de produit ingéré, notamment 23 nanomoles par kcal de produit ingéré, notamment 20 nanomoles par kcal de produit ingéré.

Selon un mode de réalisation avantageux, la présente invention concerne l'utilisation d'une composition contenant au moins un alkylglycérol, au moins un inhibiteur de la biosynthèse des polyamines, ledit inhibiteur étant un inhibiteur de l'omithine décarboxylase, un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et contenant au moins un agent antitumoral, pour la préparation d'un médicament, pour l'homme ou l'animal, destiné au traitement de pathologies tumorales,
la quantité totale de polyamines biologiquement «actives» par jour ingérées par l'homme ou l'animal, auquel le susdit médicament est administré, ne dépassant pas 30 nano moles par kcal de produit ingéré, notamment 25 nanomoles par kcal de produit ingéré, notamment 23 nanomoles par kcal de produit ingéré, notamment 20 nanomoles par kcal de produit ingéré.

Selon un mode de réalisation, la présente invention se rapporte à l'utilisation d'un produit dans lequel l'alkylglycérol peut être énantiomériquement pur, racémique ou énantiomériquement enrichi,
ledit alkylglycérol étant un dérivé du glycérol dans lequel l'une au moins des fonctions hydroxyle est alkylée par une chaîne alkyle de 10 à 30 atomes de carbone, notamment de 10 à 20 atomes de carbone, notamment de 15 à 20 atomes de carbone, saturée ou non, substituée ou non.

Le produit utilisé dans la présente invention peut également contenir un mélange d'alkylglycérols. Les alkylglycérols selon la présente invention sont préférentiellement des monoalkylglycérols, alkylés sur une des fonctions alcool primaire, énantiomères de configuration S. La formule générale de ces composés est : 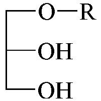 où R désigne une chaîne alkyle.

Ladite chaîne alkyle peut contenir de 1 à 30 atomes de carbones, de 10 à 20 atomes de carbones, de 10 à 15 atomes de carbones, de 15 à 20 atomes de carbones, de 5 à 25 atomes de carbones, notamment de 14 à 18 atomes de carbones. La chaîne alkyle peut être ramifiée, contenir une ou plusieurs insaturations, notamment des doubles ou triples liaisons. La chaine alkyle peut contenir des hétéroatomes, notamment de l'oxygène ou du soufre. Un, deux ou trois des atomes d'oxygène du groupement glycérol peuvent être alkylés par une chaîne carbonée. Un ou deux des atomes d'oxygène du groupement glycérol peuvent être alkylés par des chaînes carbonées, par des groupements acyles ou phosphoryles, grâce à une liaison ester.

Ladite chaîne alkyle correspond notamment aux chaînes alkyles des acides : palmitique (16 :0), stéarique (18 :0), vaccénique (18 :ln-7), linoléique (18 :2n-6), myristique (14 :0), palmitoléique (16 :1n-7), oléique (18 :ln-9), arachidique (20 :0), cis-9 eicosénoique (20 :1n-11), cis-11-eicosenoique (10 :1n-11), cis-8,11,14,17-eicosatétraénoique (22 :4n-3), arachidonique (10 :4n-6), et cis-7,10,13,16-docosatétraénoique (22 :4n-6).

Selon un mode de réalisation avantageux de l'invention la chaîne alkyle de l'alkylglycérol est notamment choisie parmi l'octadécyl-9-enyl (AKG 18 :1), l'octadécyl (AKG 18 :0), l'héxadécyl (AKG 16 :0), l'héxadécyl-9-enyl (AKG 16 :1), le tétradécyl (AKG 14 :0 ou 14 :1).

Dans le cas ou la chaîne alkyle est l'octadécyl-9-enyl, l'alkyl glycérol est nommé trivialement alcool sélachylique, numéro CAS [34043-91-9].

Le produit selon la présente invention peut également contenir des métabolites des alkylglycérols décrits ci-dessus. Le produit peut contenir un mélange d'alkylglycérols, et / ou de produits de dégradations d'alkylglycérols.

Les mélanges d'alkylglycérols les plus intéressants selon la présente invention sont les mélanges contenant notamment les alkylglycérols 18:1 et 16 :1; les mélanges d'alkylglycérols 18 :1, 18 :0 et 16 :1 ; les mélanges d'alkylglycérols 18 :1, 16 :1 et 16 :0 ; les mélanges d'alkylglycérols 18 :1, 18 :0, 16 :1 et 16 :0 ; les mélanges correspondant aux huiles de foie de requin naturelles ; les mélanges contenant notamment de 50 à 90% d'alkylglycérol C18 :1 et de 2 à 20 % d'alkylglycérol C16 :1.

Dans un mode de réalisation particulier de la présente invention, les dodécylalkylglycérol, tridécylalkylglycérol, lysophosphatidyl choline, lysophosphatidyl éthanolamine et lysophosphatidyl sérine sont exclus des alkylglycérols selon la présente invention.

Par « métabolites » on désigne les produits obtenus par métabolisation des alkylglycérols, c'est-à-dire les produits obtenus à partir d'alkylglycérols lors des réactions biochimiques endogènes ayant lieu au sein d'une cellule.

Dans un autre mode de réalisation particulier de la présente invention, les alkylglycérols endogènes naturellement présents dans l'organisme humain ou animal ou résultant, même transitoirement, d'un état pathologique sont exclus des alkylglycérols selon la présente invention.

Selon un autre mode de réalisation, la présente invention concerne l'utilisation d'un produit pour la préparation d'un médicament, lequel médicament contient moins de 30 nanomoles de polyamines biologiquement «actives» par millilitre de médicament, notamment 14 nanomoles de polyamines biologiquement «actives» par millilitre de médicament, particulièrement moins de 2 nanomoles de polyamines biologiquement «actives» par millilitre de médicament.

La divulgation concerne l'utilisation d'un produit pour la préparation d'un médicament, lequel médicament contient :
▪ moins de 400 picomoles de putrescine par gramme de médicament,
▪ moins de 400 picomoles de spermidine par gramme de médicament,
▪ moins de 400 picomoles de spermine par gramme de médicament, et
▪ moins de 400 picomoles de cadavérine par gramme de médicament.

La présente divulgation concerne l'utilisation d'un produit pour la préparation d'un médicament, lequel médicament contient moins de 23 nanomoles de polyamines biologiquement « actives » par kcal de médicament, particulièrement moins de 10 nanomoles par kcal de médicament, préférentiellement moins de 1,5 nanomoles de polyamines biologiquement « actives » par kcal de médicament.

La masse minimale de polyamines nécessaire au métabolisme d'un humain est estimée à environ 20 µg par jour (environ 0,14 µmol / j), notamment environ 10 µg par jour (environ 69 nanomoles / j), particulièrement environ 1 µg par jour (environ 7 nanomoles / j).

Selon un mode de réalisation particulier, l'invention concerne l'utilisation d'un produit, pour la préparation d'un médicament, lequel médicament contient :
▪ moins de 100 picomoles de putrescine par gramme de médicament, préférentiellement moins de 50 picomoles de putrescine par gramme de médicament,
▪ moins de 100 picomoles de spermidine par gramme de médicament, préférentiellement moins de 50 picomoles de spermidine par gramme de médicament,
▪ moins de 100 picomoles de cadavérine par gramme de médicament, préférentiellement moins de 50 picomoles de cadavérine par gramme de médicament, et
▪ moins de 100 picomoles de cadavérine par gramme de médicament, préférentiellement moins de 50 picomoles de cadavérine par gramme de médicament.

Tous les rapports de quantité à des masses de médicament sont à comprendre dans le sens de rapport à la masse de médicament sec. La masse de médicament sec correspond à la masse de médicament sans tenir compte de la masse d'eau contenue par ledit médicament.

Selon un autre mode de réalisation, la présente invention concerne l'utilisation d'un produit pour la préparation d'un médicament, lequel médicament contient en pourcentage de poids sec par rapport au poids sec total : 10% à 35% de lipides, 8% à 30% de protéines, 35% à 80% de glucides, jusqu'à 10% d'un mélange constitué de vitamines, de minéraux et d'électrolytes, et les alkylglycérols représentant plus de 2,8%, notamment plus de 6,8%, particulièrement plus de 12,8% de la masse des lipides.

Selon un autre mode de réalisation, la présente invention concerne l'utilisation d'un produit dans laquelle l'inhibiteur de la biosynthèse des polyamines est un inhibiteur de l'ornithine décarboxylase, notamment un dérivé de l'ornithine, particulièrement la 2-2'-(Difluorométhyl)ornithine (DFMO).

Le DFMO utilisé peut être sous forme racémique, énantiomériquement enrichie ou énantiomériquement pure. Le DFMO peut avoir d'autres appellations notamment éflornithine, ornidyl, 2-(Difluorométhyl)ornithine, α-Difluorométhylornithine, Vaniqa.

D'autres inhibiteurs de l'ornithine décarboxylase sont également utilisables :
- les antagonistes du phosphate de pyridoxal, notamment la L-canaline, et la N-(5'-phosphopyridoxyl)ornithine,
- les inhibiteurs compétitifs, notamment l'α-hydrazino-ornithine, l'acide DL-α-hydrazino-γ-aminovalérique, l'α-méthylornithine (a-MO), la *trans*-3-déhydro-DL-ornithine, le 1,4-diamino-*trans*-2-butène, le 1,4-diaminobutanone,
- les inhibiteurs diaminés, notamment le 1,3-diaminopropane, le 1,3-diamino-2-propanol, la bis(éthyl)spermine,
- les inhibiteurs suicides et irréversibles, notamment la monofluorométhylornithine, la 2-monofluorométhyldéhydro-ornithine, la 2-monofluorométhyldéhydro-ornithine méthyl ester, le 5-hexyne-1,4-diamine, la *trans*-hex-2-èn-5-yne-1,4-diamine, mono fluorométhylputrescine, difluorométhylputrescine, α-allénylputrescine, (2R,5R)-6-heptyne-2,5-diamine.

La présente divulgation concerne l'utilisation d'un produit pour la préparation d'un médicament, lequel médicament est destiné au traitement de pathologies
de la peau, notamment le psoriasis, la rosacée, le sarcome de Kaposi,
de l'intestin, notamment les ascites,
d'organes divers, notamment les cancers, les maladies infectieuses, les maladies auto-immunes.

La présente divulgation concerne l'utilisation d'un produit pour la préparation d'un médicament, lequel médicament est destiné au traitement de pathologies
de la peau, notamment le granulome pyogénique, les verrues, la dermatite allergique,
de l'intestin, notamment les ascites, les adhésions péritonéales,
du système reproducteur, notamment l'endométriose, la pré-éclampsie, les fausses-couches récurrentes,
des os et des articulations, notamment l'arthrite, la synovite, l'ostomyélite, la formation d'ostéophytes
de l'œil, notamment la dégénérescence maculaire liée à l'âge, la rétinopathie diabétique, la cécité,
du poumon, notamment l'hypertension pulmonaire primaire,
du tissu adipeux, notamment l'obésité dans le cas d'angiogénèses induites par les graisses,
d'organes divers, notamment, les maladies infectieuses, les maladies auto-immunes, ou
des pathologies pédiatriques, notamment l'hémangiome, la rétinopathie des prématurés.

Selon un autre mode de réalisation, la présente invention concerne l'utilisation d'un produit dans lequel l'agent antitumoral est choisi parmi les antimétabolites, les médicaments agissants sur l'ADN, les médicaments antimitotiques, les dérivés de la podophyllotoxine et les procarbazines, les inhibiteurs du VEGF et des récepteurs du VEGF, ainsi que les inhibiteurs de kinase, et notamment l'Avastin® (bevacizumab), l'Endoxan® (cyclophosphamide), le Taxotère® (docétaxel), le Cisplatine, le methotrexate, la Eldisine® (vindesine), la vincristine.

Selon un autre mode de réalisation, la présente invention concerne l'utilisation d'un produit pour la préparation d'un médicament, lequel médicament contient une quantité d'au moins un alkylglycérol correspondant à une dose journalière d'alkylglycérol(s) d'environ 0,5 ng / kcal à 4 µg / kcal, notamment environ 5 ng / kcal à 2 µg / kcal, et particulièrement environ 10 ng / kcal à 1 µg / kcal.

Selon un autre mode de réalisation, la présente invention concerne l'utilisation d'un produit, pour usage per os, pour la préparation d'un médicament, lequel médicament contient une quantité d'au moins un inhibiteur de la biosynthèse des polyamines correspondant à une dose journalière, en fonction de la masse du patient, d'inhibiteur(s) de la biosynthèse des polyamines d'environ 5 à 20 g / kg / jour, notamment 7 à 14 g / kg / jour, et particulièrement environ 9 g / kg / jour.

Selon un autre mode de réalisation, la présente invention concerne l'utilisation d'un produit, pour injection intraveineuse, pour la préparation d'un médicament, lequel médicament contient une quantité d'au moins un inhibiteur de la biosynthèse des polyamines correspondant à une dose journalière, en fonction de la masse du patient, d'inhibiteur(s) de la biosynthèse des polyamines d'environ 70 à 1 000 mg / kg / jour, notamment 400 à 800 mg / kg / jour, et particulièrement environ 600 mg / kg / jour.

Selon un autre mode de réalisation, la présente invention concerne l'utilisation d'un produit, pour la préparation d'un médicament, lequel médicament contient une quantité d'au moins un agent antitumoral correspondant à une dose journalière d'agent antitumoral d'environ 0,1 à 500 mg / m², notamment 2 à 500 mg / m², et particulièrement 5 à 200 mg / m².

Ces doses et la fréquence de leurs administrations doivent être adaptées en fonction de l'agent antitumoral utilisé. Ces valeurs sont connues de l'homme de l'art.

A titre d'exemple sont cités :
la cyclophosphamide, de 10 à 200 mg/ m²/ jour, notamment de 30 à 200 mg /m² / jour, notamment de 100 à 200 mg /m² / jour, notamment de 10 à 20 mg /m² / jour
le taxotère, de 10 à 100 mg / m²/ semaine, notamment 25 à 100 mg / m² / semaine, notamment environ 100 mg / m² / semaine,
le cisplatine, de 5 à 100 mg / m²/ mois, notamment 15 à 100 mg / m² / mois, notamment 50 à 100 mg / m² / mois, notamment de 5 à 10 mg / m²/ mois
la vincristine, de 0,1 à 2 mg / m²/ semaine, notamment 0,25 à 2 mg / m² / semaine, notamment 1 à 2 mg / m² / semaine, notamment 0,1 à 0,2 mg / m² / semaine
le methotrexate, de 1,5 à 25 mg / m²/ semaine, notamment 5 à 25 mg / m² / semaine, notamment 15 à 25 mg / m² / semaine, notamment 1,5 à 2,5 mg / m² / semaine

Selon un mode de réalisation, la présente invention concerne également un produit contenant :
- au moins un alkylglycérol,
- au moins un inhibiteur de la biosynthèse des polyamines, ledit inhibiteur étant un inhibiteur de l'omithine décarboxylase,
- un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et
- éventuellement au moins un agent antitumoral,
comme préparation de combinaison pour une utilisation simultanée, séparée ou étalée dans le temps,
la préparation de combinaison comprenant au maximum 9 mg de polyamines biologiquement « actives » par unité journalière de composition.

Tous ce qui a été décrit précédemment concernant l'utilisation de produits de combinaison et l'utilisation de compositions, notamment au sujet de la nature des composés les constituant, alkylglycérols, inhibiteurs de la biosynthèse des polyamines, agents antitumoraux et excipient appauvri en polyamine, des dosages de ces composés, de leurs conditionnements, des avantages et des contraintes liés à l'utilisation, est également applicable aux produits de combinaison et aux compositions correspondants.

Selon la présente invention la quantité de polyamines biologiquement actives pouvant être absorbée par jour par un patient ou un animal ne doit pas dépasser le seuil maximal de 30 nanomoles de polyamines biologiquement actives par kcal de préparation de combinaison ingérée, ni être inférieure au seuil minimal de environ 0,004 nanomoles de polyamines biologiquement actives par kcal de préparation de combinaison ingérée par jour. Une quantité de polyamines biologiquement actives supérieure au seuil maximal nuirait à l'efficacité de la présente invention, et une quantité de polyamines biologiquement actives inférieure au seuil minimal entrainerait des carences pouvant provoquer des troubles de la santé du patient ou de l'animal traité avec la présente invention.

Le terme « unité journalière de composition » désigne le volume total de composition absorbé durant une journée par le patient ou l'animal.

Le besoin calorique journalier d'un adulte sain est estimé à 1950 k/cal par jour.

La mise en œuvre de la présente invention implique un contrôle des apports en polyamines biologiquement actives. Selon l'invention, cet apport peut provenir du produit ou de la diète totale, c'est-à-dire l'alimentation globale du patient ou de l'animal. La diète totale inclut le produit

Afin de bien contrôler les apports en polyamines biologiquement actives, il est nécessaire de connaitre les teneurs en polyamines du produit mais également de l'ensemble des aliments constituant la diète totale.

Dans le cas où le produit constitue la diète totale, le contrôle des apports en polyamines biologiquement actives de la diète totale se confond avec le contrôle des apports du produit.

Le produit peut être présenté sous forme sèche à dissoudre extemporanément dans un véhicule neutre convenant à l'administration orale ou encore entérale, ou sous forme liquide prête à l'emploi. Le produit est avantageusement présenté sous forme stérile.

Selon un mode de réalisation avantageux, la présente invention concerne un produit contenant :
- au moins un alkylglycérol,
- au moins un inhibiteur de la biosynthèse des polyamines, ledit inhibiteur étant un inhibiteur de l'omithine décarboxylase, et
- un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine,
comme préparation de combinaison pour une utilisation simultanée, séparée ou étalée dans le temps,
ladite préparation de combinaison ne contenant pas d'agent antitumoral,
la préparation de combinaison comprenant au maximum 9 mg de polyamines biologiquement « actives » par unité journalière de composition.

Dans un mode de réalisation particulier de l'invention, deux des composants du produit de combinaison sont déjà sous forme de pré-combinaison. Ce mode de réalisation permet de contourner avantageusement les problèmes liés à la stabilité des composants, ce mode de réalisation permet également de faciliter la prise du traitement par le patient ou l'animal en réduisant le nombre de composants à absorber.

Selon un mode de réalisation avantageux, la présente invention concerne un produit contenant :
- une combinaison d'un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et d'au moins un alkylglycérol, et
- au moins un inhibiteur de la biosynthèse des polyamines, ledit inhibiteur étant un inhibiteur de l'omithine décarboxylase,
comme préparation de combinaison pour une utilisation simultanée, séparée ou étalée dans le temps,
ladite préparation de combinaison ne contenant pas d'agent antitumoral,
la préparation de combinaison comprenant au maximum 9 mg de polyamines biologiquement « actives » par unité journalière de composition.

Selon un mode de réalisation avantageux, la présente invention concerne un produit contenant :
- une combinaison d'un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et d'au moins un inhibiteur de la biosynthèse des polyamines, ledit inhibiteur étant un inhibiteur de l'omithine décarboxylase, et
- au moins un alkylglycérol,
comme préparation de combinaison pour une utilisation simultanée, séparée ou étalée dans le temps,
ladite préparation de combinaison ne contenant pas d'agent antitumoral,
la préparation de combinaison comprenant au maximum 9 mg de polyamines biologiquement « actives » par unité journalière de composition.

Selon un mode de réalisation avantageux, la présente invention concerne un produit contenant :
- une combinaison d'au moins un alkylglycérol et d'au moins un inhibiteur de la biosynthèse des polyamines, ledit inhibiteur étant un inhibiteur de l'omithine décarboxylase, et
- un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine,
comme préparation de combinaison pour une utilisation simultanée, séparée ou étalée dans le temps, ladite préparation de combinaison ne contenant pas d'agent antitumoral,
la préparation de combinaison comprenant au maximum 9 mg de polyamines biologiquement « actives » par unité journalière de composition.

Selon un mode de réalisation avantageux, la présente invention concerne un produit contenant :
- au moins un alkylglycérol,
- au moins un inhibiteur de la biosynthèse des polyamines, ledit inhibiteur étant un inhibiteur de l'omithine décarboxylase,
- un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et
- au moins un agent antitumoral,
comme préparation de combinaison pour une utilisation simultanée, séparée ou étalée dans le temps,

la préparation de combinaison comprenant au maximum 9 mg de polyamines biologiquement « actives » par unité journalière de composition.

Selon un mode de réalisation avantageux, la présente invention concerne un produit contenant :
- une combinaison d'un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et d'au moins un alkylglycérol,
- au moins un inhibiteur de la biosynthèse des polyamines, ledit inhibiteur étant un inhibiteur de l'omithine décarboxylase, et
- au moins un agent antitumoral,
comme préparation de combinaison pour une utilisation simultanée, séparée ou étalée dans le temps,
la préparation de combinaison comprenant au maximum 9 mg de polyamines biologiquement « actives » par unité journalière de composition.

Selon un mode de réalisation avantageux, la présente invention concerne un produit contenant :
- une combinaison d'au moins un inhibiteur de la biosynthèse des polyamines, ledit inhibiteur étant un inhibiteur de l'omithine décarboxylase, et d'au moins un agent antitumoral,
- au moins un alkylglycérol, et
- un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine,
comme préparation de combinaison pour une utilisation simultanée, séparée ou étalée dans le temps,
la préparation de combinaison comprenant au maximum 9 mg de polyamines biologiquement « actives » par unité journalière de composition.

Selon un mode de réalisation avantageux, la présente invention concerne un produit contenant :
- une combinaison d'un excipient appauvri en polyamines biologiquement «actives» correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et d'au moins un inhibiteur de la biosynthèse des polyamines, ledit inhibiteur étant un inhibiteur de l'omithine décarboxylase,
- au moins un alkylglycérol, et
- au moins un agent antitumoral,
comme préparation de combinaison pour une utilisation simultanée, séparée ou étalée dans le temps,

la préparation de combinaison comprenant au maximum 9 mg de polyamines biologiquement « actives » par unité journalière de composition.

Selon un mode de réalisation avantageux, la présente invention concerne un produit contenant :
- une combinaison d'au moins un alkylglycérol et d'au moins un agent antitumoral,
- au moins un inhibiteur de la biosynthèse des polyamines, ledit inhibiteur étant un inhibiteur de l'omithine décarboxylase, et
- un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine,
comme préparation de combinaison pour une utilisation simultanée, séparée ou étalée dans le temps,
la préparation de combinaison comprenant au maximum 9 mg de polyamines biologiquement « actives » par unité journalière de composition.

Selon un mode de réalisation avantageux, la présente invention concerne un produit contenant :
- une combinaison d'un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et d'au moins un agent antitumoral,
- au moins un alkylglycérol, et
- au moins un inhibiteur de la biosynthèse des polyamines, ledit inhibiteur étant un inhibiteur de l'omithine décarboxylase,
comme préparation de combinaison pour une utilisation simultanée, séparée ou étalée dans le temps,
la préparation de combinaison comprenant au maximum 9 mg de polyamines biologiquement « actives » par unité journalière de composition.

Selon un mode de réalisation avantageux, la présente invention concerne un produit contenant :
- une combinaison d'au moins un alkylglycérol et d'au moins un inhibiteur de la biosynthèse des polyamines, ledit inhibiteur étant un inhibiteur de l'omithine décarboxylase,
- d'au moins un agent antitumoral, et
- un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine,
comme préparation de combinaison pour une utilisation simultanée, séparée ou étalée dans le temps,
la préparation de combinaison comprenant au maximum 9 mg de polyamines biologiquement « actives » par unité journalière de composition.

Dans un mode de réalisation particulier de l'invention, les composants du produit de combinaison sont déjà, deux à deux, sous forme de pré-combinaison. Ce mode de réalisation permet de contourner avantageusement les problèmes liés à la stabilité des composants, ce mode de réalisation permet également de faciliter la prise du traitement par le patient ou l'animal en réduisant le nombre de composants à absorber.

Selon un mode de réalisation avantageux, la présente invention concerne un produit contenant :
- une combinaison d'un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et d'au moins un alkylglycérol, et
- une combinaison d'au moins un inhibiteur de la biosynthèse des polyamines, ledit inhibiteur étant un inhibiteur de l'omithine décarboxylase, et d'au moins un agent antitumoral,
comme préparation de combinaison pour une utilisation simultanée, séparée ou étalée dans le temps,
la préparation de combinaison comprenant au maximum 9 mg de polyamines biologiquement « actives » par unité journalière de composition.

Selon un mode de réalisation avantageux, la présente invention concerne un produit contenant :
- une combinaison d'un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et d'au moins un inhibiteur de la biosynthèse des polyamines, ledit inhibiteur étant un inhibiteur de l'omithine décarboxylase,
- une combinaison d'au moins un alkylglycérol, et d'au moins un agent antitumoral, comme préparation de combinaison pour une utilisation simultanée, séparée ou étalée dans le temps,
la préparation de combinaison comprenant au maximum 9 mg de polyamines biologiquement « actives » par unité journalière de composition.

Selon un mode de réalisation avantageux, la présente invention concerne un produit contenant :
- une combinaison d'un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et d'au moins un agent antitumoral, et
- une combinaison d'au moins un alkylglycérol et d'au moins un inhibiteur de la biosynthèse des polyamines, ledit inhibiteur étant un inhibiteur de l'omithine décarboxylase, comme préparation de combinaison pour une utilisation simultanée, séparée ou étalée dans le temps,
la préparation de combinaison comprenant au maximum 9 mg de polyamines biologiquement « actives » par unité journalière de composition.

Dans un mode de réalisation particulier de l'invention, trois des composants du produit de combinaison sont déjà sous forme de pré-combinaison. Ce mode de réalisation permet de contourner avantageusement les problèmes liés à la stabilité des composants, ce mode de réalisation permet également de faciliter la prise du traitement par le patient ou l'animal en réduisant le nombre de composants à absorber.

Selon un mode de réalisation avantageux, la présente invention concerne un produit contenant :
- une combinaison d'un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, d'au moins un alkylglycérol et d'au moins un inhibiteur de la biosynthèse des polyamines, ledit inhibiteur étant un inhibiteur de l'omithine décarboxylase, et
- au moins un agent antitumoral comme préparation de combinaison pour une utilisation simultanée, séparée ou étalée dans le temps,
la préparation de combinaison comprenant au maximum 9 mg de polyamines biologiquement « actives » par unité journalière de composition.

Selon un mode de réalisation avantageux, la présente invention concerne un produit contenant :
- une combinaison d'au moins un alkylglycérol, d'au moins un agent antitumoral, et d'au moins un inhibiteur de la biosynthèse des polyamines, ledit inhibiteur étant un inhibiteur de l'omithine décarboxylase, et
- un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine,
comme préparation de combinaison pour une utilisation simultanée, séparée ou étalée dans le temps,
la préparation de combinaison comprenant au maximum 9 mg de polyamines biologiquement « actives » par unité journalière de composition.

Selon un mode de réalisation avantageux, la présente invention concerne un produit contenant :
- une combinaison d'un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, d'au moins un agent antitumoral, et d'au moins un inhibiteur de la biosynthèse des polyamines, ledit inhibiteur étant un inhibiteur de l'omithine décarboxylase, et
- d'au moins un alkylglycérol,
comme préparation de combinaison pour une utilisation simultanée, séparée ou étalée dans le temps,
la préparation de combinaison comprenant au maximum 9 mg de polyamines biologiquement « actives » par unité journalière de composition.

Selon un mode de réalisation avantageux, la présente invention concerne un produit contenant :
- une combinaison d'un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, d'au moins un agent antitumoral, et d'au moins un alkylglycérol, et
- d'au moins un inhibiteur de la biosynthèse des polyamines, ledit inhibiteur étant un inhibiteur de l'omithine décarboxylase,
comme préparation de combinaison pour une utilisation simultanée, séparée ou étalée dans le temps,
la préparation de combinaison comprenant au maximum 9 mg de polyamines biologiquement « actives » par unité journalière de composition.

Selon un mode de réalisation avantageux, la présente invention concerne une composition contenant au moins un alkylglycérol, au moins un inhibiteur de la biosynthèse des polyamines, ledit inhibiteur étant un inhibiteur de l'omithine décarboxylase, un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et contenant au moins un agent antitumoral,
la composition comprenant au maximum 9 mg de polyamines biologiquement « actives » par unité journalière de composition.

Selon un autre mode de réalisation, la présente invention concerne un produit contenant moins de 30 nanomoles de polyamines biologiquement «actives» par kcal de produit, notamment 14 nanomoles de polyamines biologiquement «actives» par kcal de produit, particulièrement moins de 2 nanomoles de polyamines biologiquement «actives» par kcal de produit.

Tous les rapports de quantité à des masses de composition sont à comprendre dans le sens de rapport à la masse de composition sèche. La masse de produit sec correspond à la masse de produit sans tenir compte de la masse d'eau contenue par ledit produit.

Selon un autre mode de réalisation, la présente invention concerne un produit, lequel produit comprend au minimum 20 µg, notamment 10 µg, notamment 1 µg de polyamines biologiquement « actives » par unité journalière de produit.

Selon un mode de réalisation particulier, la présente invention concerne un produit contenant :
▪ moins de 400 picomoles de putréscine par gramme de produit,
▪ moins de 400 picomoles de spermidine par gramme de produit,
▪ moins de 400 picomoles de spermine par gramme de produit, et
▪ moins de 400 picomoles de cadavérine par gramme de produit.

Selon un mode de réalisation particulier, la présente invention concerne un produit contenant moins de 23 nanomoles de polyamines biologiquement « actives » par kcal de produit, particulièrement moins de 10 nanomoles par kcal de produit, préférentiellement au moins 1,5 nanomoles de polyamines biologiquement « actives » par kcal de produit.

Selon un mode de réalisation particulier, la présente invention concerne un produit contenant :
▪ moins de 100 picomoles de putréscine par gramme de produit, préférentiellement moins de 50 picomoles de putréscine par gramme de produit,
▪ moins de 100 picomoles de spermidine par gramme de produit, préférentiellement moins de 50 picomoles de spermidine par gramme de produit,
▪ moins de 100 picomoles de cadavérine par gramme de produit, préférentiellement moins de 50 picomoles de cadavérine par gramme de produit, et
▪ moins de 100 picomoles de cadavérine par gramme de produit, préférentiellement moins de 50 picomoles de cadavérine par gramme de produit.

Selon un mode de réalisation particulier, la présente invention concerne une préparation de combinaison décrite précédemment, pour une utilisation comme médicament pour le traitement de pathologies tumorales.

Selon un mode de réalisation particulier, la présente invention concerne une préparation de combinaison décrite précédemment, pour le traitement de pathologies tumorales.

Selon un mode de réalisation particulier, la présente invention concerne un produit, contenant une quantité d'au moins un alkylglycérol correspondant à une dose journalière d'alkylglycérol(s) d'environ 0,5 ng / kcal à 4 µg / kcal, notamment environ 5 ng / kcal à 2 µg / kcal, et particulièrement environ 10 ng / kcal à 1 µg / kcal.

Selon un mode de réalisation particulier, la présente invention concerne un produit, pour usage per os, contenant une quantité, en fonction de la masse du patient, d'au moins un inhibiteur de la biosynthèse des polyamines correspondant à une dose journalière d'inhibiteur(s) de la biosynthèse des polyamines d'environ 5 à 20 g / kg / jour, notamment 7 à 14 g / kg / jour, et particulièrement environ 9 g / kg / jour.

Selon un mode de réalisation particulier, la présente invention concerne un produit, pour injection intraveineuse, contenant une quantité d'au moins un inhibiteur de la biosynthèse des polyamines correspondant à une dose journalière, en fonction de la masse du patient, d'inhibiteur(s) de la biosynthèse des polyamines d'environ 70 à 1 000 mg / kg / jour, notamment 400 à 800 mg / kg / jour, et particulièrement environ 600 mg / kg / jour.

Selon un mode de réalisation particulier, la présente invention concerne un produit, contenant une quantité d'au moins un agent antitumoral correspondant à une dose journalière d'agent antitumoral d'environ 0,1 à 500 mg / m², notamment 2 à 500 mg / m², et particulièrement 5 à 200 mg / m².

Ces doses et la fréquence de leurs administrations doivent être adaptées en fonction de l'agent antitumoral utilisé. Ces valeurs sont connues de l'homme de l'art.

A titre d'exemple sont cités :
la cyclophosphamide, de 10 à 200 mg/ m²/ jour, notamment de 30 à 200 mg /m² / jour, notamment de 100 à 200 mg /m² / jour, notamment de 10 à 20 mg /m² / jour
le taxotère, de 10 à 100 mg / m²/ semaine, notamment 25 à 100 mg / m² / semaine, notamment environ 100 mg / m² / semaine,
le cisplatine, de 5 à 100 mg / m²/ mois, notamment 15 à 100 mg / m² / mois, notamment 50 à 100 mg / m² / mois, notamment de 5 à 10 mg / m²/ mois
la vincristine, de 0,1 à 2 mg / m²/ semaine, notamment 0,25 à 2 mg / m² / semaine, notamment 1 à 2 mg / m² / semaine, notamment 0,1 à 0,2 mg / m² / semaine
le methotrexate, de 1,5 à 25 mg / m²/ semaine, notamment 5 à 25 mg / m² / semaine, notamment 15 à 25 mg / m² / semaine, notamment 1,5 à 2,5 mg / m² / semaine

Selon un mode de réalisation particulier, la présente invention concerne un produit, contenant en pourcentage de poids sec par rapport au poids sec total : 10% à 35% de lipides, 8% à 30% de protéines, 35% à 80% de glucides, jusqu'à 10% d'un mélange constitué de vitamines, de minéraux et d'électrolytes, et les alkylglycérols représentant plus de 2,8%, notamment plus de 6,8%, particulièrement plus de 12,8% de la masse des lipides

Selon un mode de réalisation préféré, la présente invention concerne un produit contenant :
- de 10 ng / kcal à 1 µg / kcal de l'alkylglycérol C18 :1,
- environ 9 mg de DFMO,
- un excipient contenant de 1 µg à 9 mg de polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et
- éventuellement de 10 mg à 200 mg de cyclophosphamide,
comme préparation de combinaison pour une utilisation simultanée, séparée ou étalée dans le temps,
Selon un mode de réalisation préféré, la présente invention concerne un produit contenant :
- de 10 ng / kcal à 5 µg / kcal de mélange naturel d'alkylglycérol, ou de 10 ng/kcal à 1 µg / kcal de l'alkylglycérol C18 :1,
- environ 9 mg de DFMO ou d'alpha-méthylornithine,
- un excipient contenant de 1 µg à 9 mg de polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et
- éventuellement de 10 mg à 200 mg de cyclophosphamide, comme préparation de combinaison pour une utilisation simultanée, séparée ou étalée dans le temps,

Selon un mode de réalisation préféré, la présente invention concerne un produit contenant :
- de 10 ng / kcal à 5 µg / kcal de mélange naturel d'alkylglycérol, ou de 10 ng/kcal à 1 µg / kcal de l'alkylglycérol C18 :1,
- environ 5 mg d'alpha-méthylornithine,
- un excipient contenant de 1 µg à 9 mg de polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et
- éventuellement de 10 mg à 200 mg de cyclophosphamide,
comme préparation de combinaison pour une utilisation simultanée, séparée ou étalée dans le temps,
la préparation de combinaison comprenant au maximum 9 mg de polyamines biologiquement « actives » par unité journalière de composition.

La préparation de combinaison selon la présente invention peut se présenter sous forme solide, c'est-à-dire sous forme de poudre, comprimé, pastille, gélule ; destiné à être administré directement ou dissous dans un liquide.

La préparation de combinaison selon l'invention peut également se présenter sous forme liquide, dans un conditionnement destiné à être administré directement, ou sous une forme concentrée destinée à être diluée avant administration.

Dans un mode de réalisation préféré, la préparation de combinaison selon l'invention se présente sous la forme d'un liquide prêt à être ingérée.

Dans un autre mode de réalisation préféré, la préparation de combinaison selon l'invention se présente sous la forme d'une poudre ; après ajout d'un liquide, ladite poudre permet de constituer une solution, ou une suspension, destinée à être ingérée.

la préparation de combinaison selon la présente invention peut éventuellement être mélangée à un aliment solide, semi-solide, ou à un liquide.

la préparation de combinaison selon la présente invention peut entrer dans la préparation d'une composition nutraceutique servant de substitut alimentaire, ou de complément alimentaire, destinée à un patient ou à un animal, souffrant de dysfonctionnement de l'angiogénèse et / ou de tumeurs.

Par « substitut alimentaire » on désigne une composition consommée à la place de toute autre forme d'alimentation. Un substitut alimentaire est destiné à remplacer une alimentation normale tout en assurant l'apport quotidien en nutriments. Ce type de régime alimentaire est qualifié de régime strict.

Il convient de noter que dans le cas d'un régime strict, toutes les polyamines biologiquement actives absorbées quotidiennement proviennent de la composition nutraceutique obtenu à partir de la préparation de combinaison selon l'invention. Ce mode de préparation d'une composition nutraceutique permet de contrôler avantageusement les apports quotidiens en polyamines du patient ou de l'animal.

Par « complément alimentaire » on désigne une composition consommée en plus de l'alimentation normale. Un complément alimentaire s'ajoute au régime alimentaire normal. Le régime alimentaire normal peut être modifié pour tenir compte des apports du complément alimentaire afin de ne pas perturber l'équilibre alimentaire. Par modification du régime alimentaire normal on désigne une réduction des quantités de un, plusieurs ou tous les aliments, ou une modification de la nature des aliments absorbés.

La composition nutraceutique obtenu à partir de la préparation de combinaison selon la présente invention peut-être la seule source d'alimentation, ou une source d'alimentation utilisée en complément d'une autre source d'alimentation, sans entrainer de carences chez le patient ou l'animal.

Par « autre source d'alimentation » on désigne toutes formes de régime alimentaire, c'est-à-dire l'ensemble des aliments pouvant constituer l'alimentation d'un patient ou animal, un régime alimentaire composé de substituts de repas, ou tout autre source de nourriture permettant de maintenir le patient ou l'animal en vie. En évitant les aliments contenant des polyamines

Par « carences » on désigne un manque en nutriments pouvant altérer la condition physique ou mentale d'un patient ou d'un animal.

La présente invention implique un contrôle des apports en polyamines biologiquement actives. Selon l'invention, cet apport peut provenir de la préparation de combinaison ou de la diète totale, c'est-à-dire l'alimentation globale du patient ou de l'animal. La diète totale inclut la préparation de combinaison.

Dans le cas ou la composition nutraceutique constitue la diète totale, le contrôle des apports en polyamines biologiquement actives de la diète totale se confond avec le contrôle des apports de la composition nutraceutique.

Dans le cas ou la composition nutraceutique ne constitue pas la diète totale, le contrôle des apports en polyamines biologiquement actives comprend deux niveaux distincts : la diète totale, et la préparation de combinaison. La présente invention implique un contrôle de ces deux niveaux.

### Description des figures

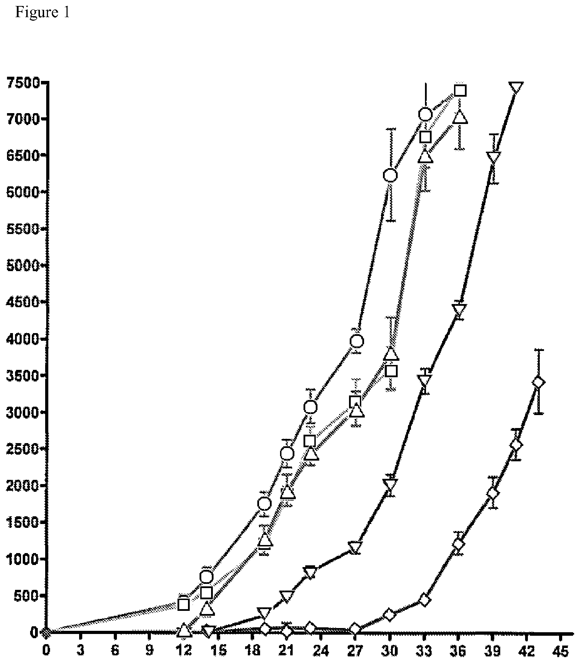

### Figure 1 : Survie et évolution du volume tumoral

Cette figure représente les variations de volume de tumeurs (en mm³, indiqués en ordonnées) en fonction du nombre de jours (indiqués en abscisses) écoulés après la greffe de ladite tumeur chez des souris C57BL/6. Les souris sont greffées avec une tumeur solide de cellules du carcinome pulmonaire de Lewis.

Cinq groupes de souris sont alimentés avec des régimes alimentaires différents.

La courbe matérialisée par des cercles représente les souris alimentées avec un régime contenant un taux normal de polyamines (128 mg/ kg d'aliment), et enrichi en huile d'olive (20 g/ kg d'aliment).

La courbe matérialisée par des carrés représente les souris alimentées avec un régime contenant un taux normal de polyamines (128 mg/ kg d'aliment), et enrichi en alkylglycérol 18 :1 (alcool sélachylique) (25 mg / souris / jour).

La courbe matérialisée par des triangles (pointant vers le haut) représente les souris alimentées avec un régime appauvri en polyamines (10 µg/ kg d'aliment), et enrichi en alkylglycérol 18 :1 (25 mg / souris / jour).

La courbe matérialisée par des triangles (pointant vers le bas) représente les souris alimentées avec un régime appauvri en polyamines (10 µg/ kg d'aliment), enrichi en huile d'olive, et enrichi en DFMO (a-difluorométhylornithine) (30 g / L).

La courbe matérialisée par des losanges représente les souris alimentées avec un régime appauvri en polyamines (10 µg/ kg d'aliment), enrichi en alkylglycérol 18 :1 (25 mg / souris / jour) et enrichi en DFMO (a-difluorométhylornithine) (30 g / L).

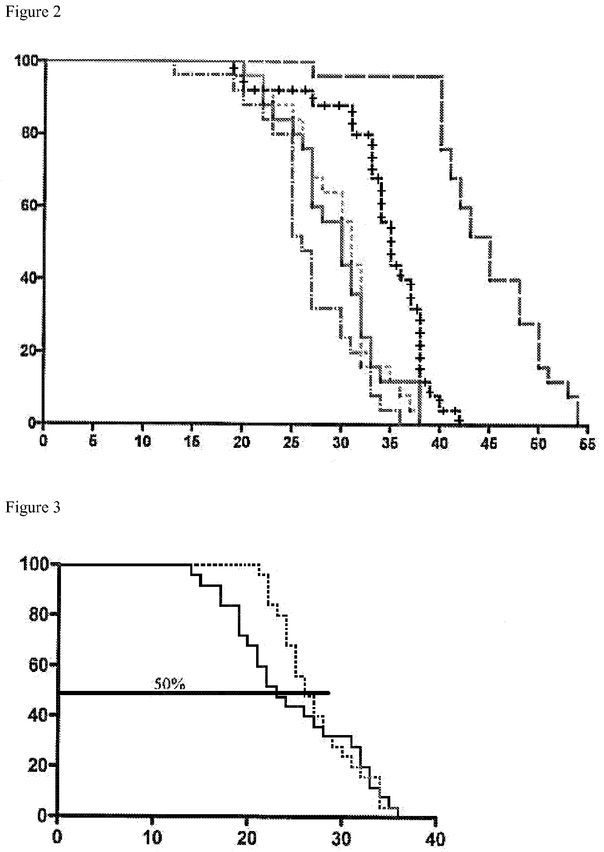

### Figure 2 Evolution de la survie

Cette figure représente le pourcentage de survie (en ordonnées) de souris C57BL/6, en fonction du nombre de jours (en abscisses) écoulés après la greffe d'une tumeur solide de cellules du carcinome pulmonaire de Lewis. La représentation utilisée est sous forme de graphique de Kaplan-Meier, le test statistique Log-Rank est utilisé pour la comparaison de la survie (fiabilité p < 0,0001).

Cinq groupes de souris sont alimentés avec des régimes alimentaires différents.

Le premier groupe (représenté par une alternance de traits long et courts: - · -) est alimenté avec un régime contenant un taux normal de polyamines (128 mg/ kg d'aliment), et enrichi en huile d'olive (20 g / kg d'aliment). La valeur médiane de survie des souris de ce groupe est de 26 jours.

Le second groupe (représenté par les pointillés de traits courts égaux : ···) est alimenté avec un régime contenant un taux normal de polyamines, et enrichi en alkylglycérol 18 :1 (alcool sélachylique) (25 mg / souris / jour). La valeur médiane de survie des souris de ce groupe est de 31 jours.

Le troisième groupe (représenté par le trait continu) est alimenté avec un régime appauvri en polyamines, et enrichi en alkylglycérol 18 :1 (alcool sélachylique) (25 mg / souris / jour). La valeur médiane de survie des souris de ce groupe est de 30 jours.

Le quatrième groupe (représenté par les signes plus : + + +) est alimenté avec un régime appauvri en polyamines, et enrichi en huile d'olive et en DFMO (a-difluorométhylornithine) (30 g / L). La valeur médiane de survie des souris de ce groupe est de 35 jours.

Le cinquième groupe (représenté par les pointillés de traits de longueurs égales : - - -) est alimenté avec un régime appauvri en polyamines, et enrichi en alkylglycérol 18 :1 (alcool sélachylique) (25 mg / souris / jour) et en DFMO (a-difluorométhylornithine) (30 g / L). La valeur médiane de survie des souris de ce groupe est de 45 jours.

### Figure 3 Etude de survie Comparaison entre l'huile d'olive (appauvri en polyamines) et C18 :1 (appauvri en polyamines)

Cette figure représente le pourcentage de survie (en ordonnées) de souris, en fonction du nombre de jours (en abscisses) écoulés après la greffe d'une tumeur solide de cellules du carcinome pulmonaire de Lewis. Les souris sont réparties en 2 groupes de 25 individus. Les deux groupes sont alimentés avec des régimes alimentaires différents. Les valeurs médianes de survie sont observées. La représentation utilisée est sous forme de graphique de Kaplan-Meier, le test statistique Log-Rank est utilisé pour la comparaison de la survie.

Le premier groupe (représenté par un trait continu) est alimenté avec un régime appauvri en polyamines (10 µg / kg d'aliment), et enrichi en huile d'olive (20 mg / kg d'aliment). La valeur médiane de survie des souris de ce groupe est de 23 jours.

Le second groupe (représenté par un pointillé) est alimenté avec un régime appauvri en polyamines (10 µg / kg d'aliment), et enrichi en alkylglycérol 18 :1 (alcool sélachylique) (25 mg / souris / jour). La valeur médiane de survie des souris de ce groupe est de 26 jours.

Aucune différence significative dans la valeur de survie médiane n'est observée (différence p = 0.5, Ratio = 0,88, χ²=0,45 (ddl=1)).

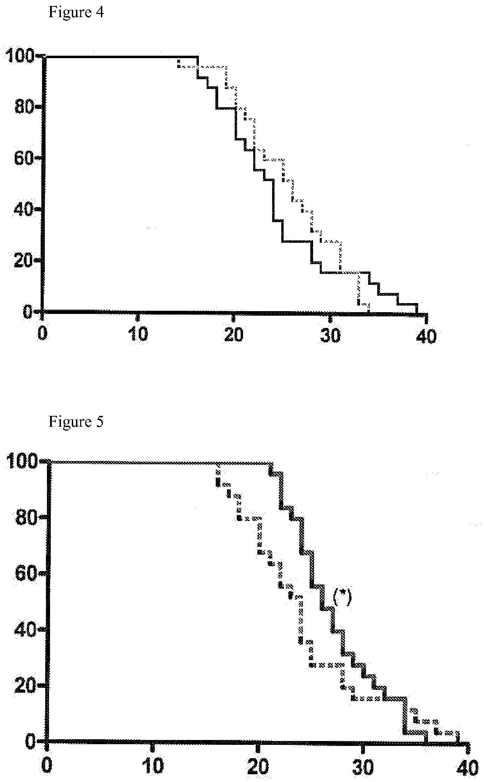

### Figure 4 Etude de survie Comparaison entre l'huile d'olive (avec polyamines) et et C18 :1 (avec polyamines)

Cette figure représente le pourcentage de survie (en ordonnées) de souris, en fonction du nombre de jours (en abscisses) écoulés après la greffe d'une tumeur solide de cellules du carcinome pulmonaire de Lewis. Les souris sont réparties en 2 groupes de 25 individus. Les deux groupes sont alimentés avec des régimes alimentaires différents. Les valeurs médianes de survie sont observées. La représentation utilisée est sous forme de graphique de Kaplan-Meier, le test statistique Log-Rank est utilisé pour la comparaison de la survie.

Le premier groupe (représenté par un trait continu) est alimenté avec un régime contenant des polyamines (128 mg / kg d'aliment), et enrichi en huile d'olive (20 g / kg d'aliment). La valeur médiane de survie des souris de ce groupe est de 24 jours.

Le second groupe (représenté par un pointillé) est alimenté avec un régime contenant des polyamines (128 mg / kg d'aliment), et enrichi en alkylglycérol 18 :1 (25 mg / souris / jour). La valeur médiane de survie des souris de ce groupe est de 26 jours.

Aucune différence significative dans la valeur de survie médiane n'est observée (différence p = 0,86, Ratio = 0,92, χ²=0,03 (ddl=1)).

### Figure 5 Etude de survie Comparaison entre l'huile d'olive (avec polyamines) et C18 :1 (appauvri en polyamines)

Cette figure représente le pourcentage de survie (en ordonnées) de souris, en fonction du nombre de jours (en abscisses) écoulés après la greffe d'une tumeur solide de cellules du carcinome pulmonaire de Lewis. Les souris sont réparties en 2 groupes de 25 individus. Les deux groupes sont alimentés avec des régimes alimentaires différents. Les valeurs médianes de survie sont observées. La représentation utilisée est sous forme de graphique de Kaplan-Meier, le test statistique Log-Rank est utilisé pour la comparaison de la survie.

Le premier groupe (représenté par un pointillé) est alimenté avec un régime alimentaire contenant des polyamines (128 mg / kg d'aliment), et enrichi en huile d'olive (20 g / kg d'aliment). La valeur médiane de survie des souris de ce groupe est de 24 jours.

Le second groupe (représenté par un trait continu) est alimenté avec un régime appauvri en polyamines (10 µg / kg d'aliment), et enrichi en alkylglycérol 18 :1 (25 mg / souris / jour). La valeur médiane de survie des souris de ce groupe est de 26 jours.

Aucune différence significative dans la valeur de survie médiane n'est observée (différence p = 0,39, Ratio = 0,92, χ²=0,73 (ddl=1)).

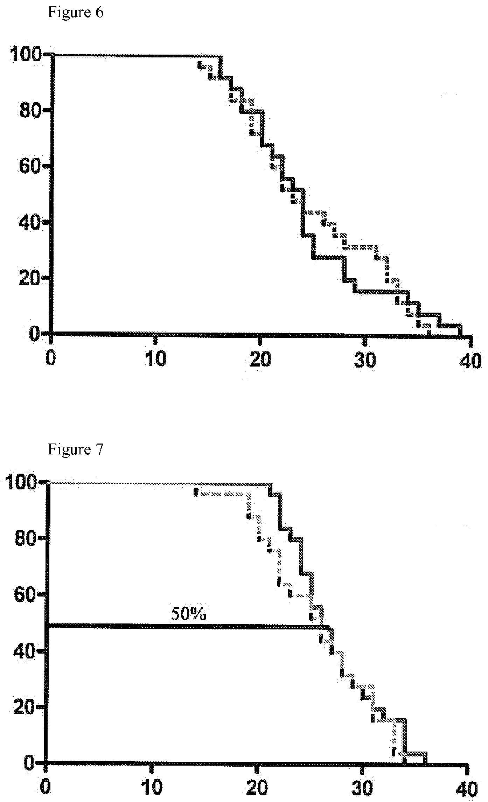

### Figure 6 Etude de survie Comparaison entre l'huile d'olive (avec polyamines) et l'huile d'olive (appauvri en polyamines)

Cette figure représente le pourcentage de survie (en ordonnées) de souris, en fonction du nombre de jours (en abscisses) écoulés après la greffe d'une tumeur solide de cellules du carcinome pulmonaire de Lewis. Les souris sont réparties en 2 groupes de 25 individus. Les deux groupes sont alimentés avec des régimes alimentaires différents. Les valeurs médianes de survie sont observées. La représentation utilisée est sous forme de graphique de Kaplan-Meier, le test statistique Log-Rank est utilisé pour la comparaison de la survie.

Le premier groupe (représenté par un pointillé) est alimenté avec un régime appauvri polyamines (10 µg / kg d'aliment), et enrichi en huile d'olive (20 g / kg d'aliment). La valeur médiane de survie des souris de ce groupe est de 23 jours.

Le second groupe (représenté par un trait continu) est alimenté avec un régime contenant des polyamines (128 mg / kg d'aliment), et enrichi en huile d'olive (20 g / kg d'aliment). La valeur médiane de survie des souris de ce groupe est de 27 jours.

Aucune différence significative dans la valeur de survie médiane n'est observée (différence p = 0,83, Ratio = 0,95, χ²=0,04 (ddl=1)).

### Figure 7 Etude de survie Comparaison entre C 18 :1 (appauvri en polyamines) et C18 :1 (avec polyamines)

Cette figure représente le pourcentage de survie (en ordonnées) de souris, en fonction du nombre de jours (en abscisses) écoulés après la greffe d'une tumeur solide de cellules du carcinome pulmonaire de Lewis. Les souris sont réparties en 2 groupes de 25 individus. Les deux groupes sont alimentés avec des régimes alimentaires différents. Les valeurs médianes de survie sont observées. La représentation utilisée est sous forme de graphique de Kaplan-Meier, le test statistique Log-Rank est utilisé pour la comparaison de la survie.

Le premier groupe (représenté par un trait continu) est alimenté avec un régime appauvri polyamines (10 µg / kg d'aliment), et enrichi en alkylglycérol 18 :1 (25 mg / souris / jour). La valeur médiane de survie des souris de ce groupe est de 26 jours.

Le second groupe (représenté par un pointillé) est alimenté avec un régime contenant des polyamines (128 mg / kg d'aliment), et enrichi en alkylglycérol 18 :1 (25 mg / souris / jour). La valeur médiane de survie des souris de ce groupe est de 26 jours.

Aucune différence significative dans la valeur de survie médiane n'est observée (différence p = 0,37, Ratio = 1, χ²=0,77 (ddl=l)).

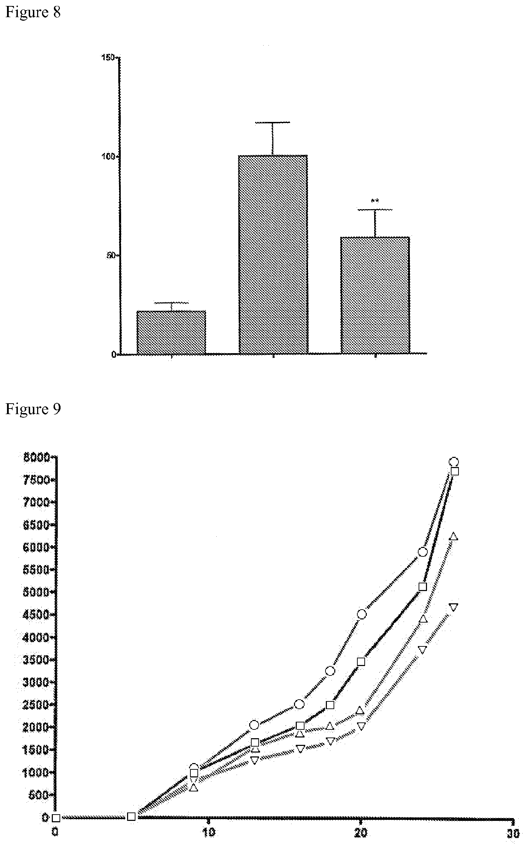

### Figure 8 Détection des effets antiangiogéniques du C18 :1

Cette figure représente le pourcentage de concentration en hémoglobine dans du Matrigel (BD Biosciences) (en ordonnées), 10 jours après injection dudit Matrigel (500µL) à des souris.

Trois groupes de souris sont composés, ces groupes sont représentés par trois colonnes.

La colonne du centre représente les souris témoins ayant été traitées avec du VEGF. Ces souris ont été greffées avec du Matrigel contenant du VEGF (50 ng/mL). Le pourcentage de concentration en hémoglobine est fixé arbitrairement à 100% et sert de référence pour quantifier les pourcentages des deux autres groupes.

La colonne de gauche représente le groupe de souris témoin. Ces souris ont été greffées avec le Matrigel, le pourcentage de concentration en hémoglobine est de 21,63%, par rapport à la colonne du centre.

La colonne de droite représente les souris ayant été greffées avec du matrigel contenant du VEGF (50 ng/mL) et alimentées par une alimentation enrichie en C18 :1.

Le pourcentage de concentration en hémoglobine est de 58,56% par rapport à la colonne du centre (** test non paramétrique de Mann et Withney : p =0,0086).

### Figure 9 Association C18 :1 avec cyclophosphamide Volume de tumeur

Cette figure représente les variations de volume de tumeurs (en mm³, indiqués en ordonnées) en fonction du nombre de jours (indiqués en abscisses) écoulés après la greffe de ladite tumeur chez des souris. Les souris sont greffées avec une tumeur solide de cellules du carcinome pulmonaire de Lewis.

Quatre groupes de souris sont alimentées avec des régimes alimentaires différents. Tous les régimes alimentaires sont appauvris en polyamines (10 µg / kg d'aliment).

La courbe matérialisée par des cercles représente les souris ayant un régime enrichi en huile d'olive (20 g/ kg d'aliment).

La courbe matérialisée par des carrés représente les souris ayant un régime enrichi en huile d'olive 20 g/ kg d'aliment) et cyclophosphamide (100 µL par souris, 20 mg/kg, 1 fois par semaine en intra péritonéal).

La courbe matérialisée par des triangles pointant vers le haut représente les souris ayant un régime enrichi en alkylglycérol C18 :1 (alcoll sélachylique) (25mg/souris/jour).

La courbe matérialisée par des triangles pointant vers le bas représente les souris ayant un régime enrichi en alkylglycérol C18 :1 (25mg/souris/jour), et cyclophosphamide (100 µL par souris, 20 mg/kg, 1 fois par semaine en intra péritonéal).

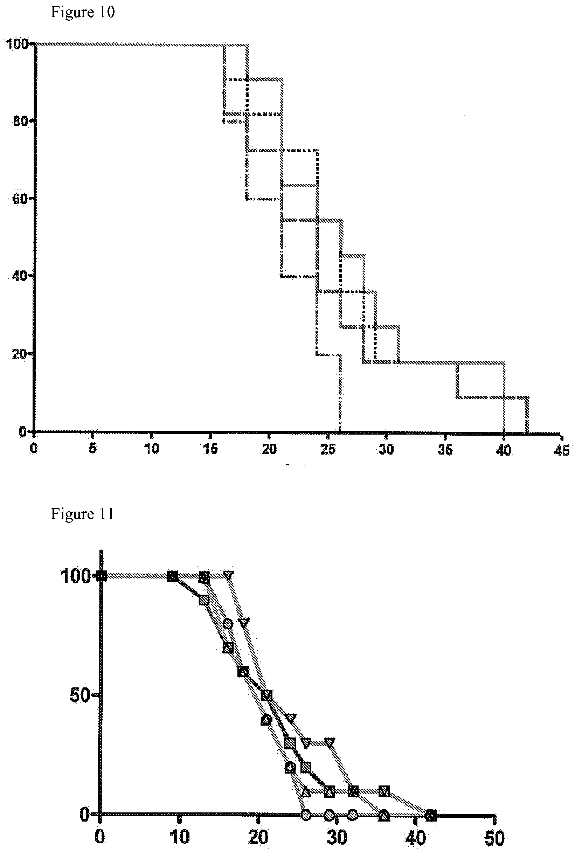

### Figure 10 Association C18 :1 avec cyclophosphamide Proportion de survie

Cette figure représente le pourcentage de survie (en ordonnées) de souris, en fonction du nombre de jours (en abscisses) écoulés après la greffe d'une tumeur solide de cellules du carcinome pulmonaire de Lewis. Les souris sont réparties en 4 groupes, alimentées avec des régimes alimentaires différents. Tous les régimes alimentaires sont appauvri en polyamines (10 µg / kg d'aliment).

Les valeurs médianes de survie sont observées. La représentation utilisée est sous forme de graphique de Kaplan-Meier, le test statistique Log-Rank est utilisé pour la comparaison de la survie.

Le premier groupe (représenté par une alternance de traits long et courts: - · -) est alimenté avec un régime enrichi en huile d'olive (20 g / kg d'aliment).

Le second groupe (représenté par les pointillés de traits courts égaux : ···) est alimenté avec un régime enrichi en huile d'olive (20 g / kg d'aliment) et cyclophosphamide (100 µL par souris, 20 mg/kg, 1 fois par semaine en intra péritonéal).

Le troisième groupe (représenté par les pointillés de traits de longueurs égales : ---) est alimenté avec un régime enrichi en alkylglycérol C18 :1 (alcool sélachylique) (25mg/souris/jour).

Le quatrième groupe (représenté par le trait continu) est alimenté avec un régime enrichi en alkylglycérol C18 :1 (25mg/souris/jour), et cyclophosphamide (100 µL par souris, 20 mg/kg, 1 fois par semaine en intra péritonéal).

### Figure 11 Association C18 :1 avec cyclophosphamide pourcentage de survie moyen

Cette figure représente le pourcentage de survie (en ordonnées) de souris, en fonction du nombre de jours (en abscisses) écoulés après la greffe d'une tumeur solide de cellules du carcinome pulmonaire de Lewis.

Quatre groupes de souris sont alimentées avec des régimes alimentaires différents. Tous les régimes alimentaires sont appauvris en polyamines (10 µg / kg d'aliment).

La courbe matérialisée par des cercles représente les souris ayant un régime enrichi en huile d'olive (20 g / kg d'aliment).

La courbe matérialisée par des carrés représente les souris ayant un régime enrichi en huile d'olive (20 g / kg d'aliment) et cyclophosphamide (100 µL par souris, 20 mg/kg, 1 fois par semaine en intra péritonéal).

La courbe matérialisée par des triangles pointant vers le haut représente les souris ayant un régime enrichi en alkylglycérol C18 :1 (alcool sélachylique) (25mg/souris/jour).

La courbe matérialisée par des triangles pointant vers le bas représente les souris ayant un régime enrichi en alkylglycérol C18 :1 (25mg/souris/jour), et cyclophosphamide (100 µL par souris, 20 mg/kg, 1 fois par semaine en intra péritonéal).

### Partie expérimentale

### Modèle animal

Des souris femelles C57BL/6, âgées de 8 semaines et pesant 20g (élevage Janvier, Le Genest St Isle, France) sont acclimatées 1 semaine avant le début de l'étude. La manipulation des animaux est réalisée en accord avec les directives éthiques pour l'expérimentation et selon les recommandations de l'Association Européenne de Recherche Biomédicale.

Les animaux sont enfermés à raison de 25 individus par cage dans des conditions standardisées (21 ± 1°C; 60% d'humidité relative, cycles de 12h de lumière, 12h d'obscurité), et ont accès à de la nourriture et à de l'eau à volonté.

Tous les produits chimiques ont été obtenus chez Baker Chemicals (Deventer, Pays-Bas) ou Merck (Darmstadt, Allemagne). La D,L-2-(difluorométhyl)ornithine (DFMO) a été donnée par le Centre de Recherche Merrel International (67000 Strasbourg, France)

### Alimentation

La nourriture synthétique appauvrie en polyamines est préparée à base de composés de Sigma Chemical Co. (St. Louis, MO, USA), selon la formulation de L. Chamaillard (Thèse n°1466, soutenue le 13 juin 1993, « Effet antitumoral d'une carence en polyamine, et ses conséquences sur le système de défense de l'organisme cancéreux », Université de Rennes 1, France) (PDD, Polyamine Deficient Diet). Cette nourriture appauvrie en polyamines contient moins de 10 µg/kg de polyamines.

La nourriture synthétique contenant des polyamines (PCD Polyamine Containing Diet) est préparée en mélangeant 128 mg de polyamines par kilo à la nourriture appauvrie en polyamines précédemment décrite (PDD). Cette quantité de polyamines correspond à celle présente dans un aliment standard pour rongeur (Charles Rivers, Villemoisson-sur-Orges, France), qui contient 128 mg de polyamines par kilo.

Les produits sont distribués aux animaux selon leur profil et leur besoin nutritionnel. Une souris consomme environ 2 grammes par jour d'alimentation.

### I Cancer

### Modèle tumoral

Les cellules de carcinome pulmonaire de Lewis (3LL) ont été obtenues auprès de l'ECACC (European collection of cell cultures) et greffées par injection intramusculaire dans les pattes postérieures de souris femelles C57BL/6 selon la méthode décrite précédemment (Hergueux J. et al., Exp. Cell Biol., 1983, 51(4), 181-191) pour produire des cellules tumorales. Après 20 jours, les cellules tumorales sont collectées et dispersées dans du PBS, comptées et diluées pour atteindre 1 ×10⁶ cellules / 100 µL, avant d'être greffées dans les souris.

### 1 Effet d'un régime appauvri en polyamines

Les produits sont distribués aux animaux selon leur profil et leur besoin nutritionnel.

Au premier jour de l'expérience, 0,1 ×10⁶ cellules tumorales sont greffées par injection intramusculaire, à chaque souris. Les souris greffées sont réparties aléatoirement dans les cages par groupe de 25 individus.

La moitié des souris est nourrie avec l'aliment appauvri en polyamine (PDD), l'autre moitié avec l'aliment contenant des polyamines (PCD).

La croissance de la tumeur est quantifiée tous les deux jours par mesure du volume de la tumeur, selon la méthode décrite précédemment (Moulinoux et al., Int J. Cancer, 1984, 34(2), 277-281).

Les animaux sont sacrifiés 20 jours après la greffe de tumeur.

### 2 Etude de survie en fonction du régime alimentaire et de l'alkylglycérol

Quatre groupes sont constitués, chacun comprenant 25 souris greffées avec des cellules tumorales. Les souris sont réparties dans 8 cages avec 12 à 13 individus par cage. Chaque groupe de souris comprend les individus de deux cages. Chaque groupe de souris est alimenté avec un régime alimentaire différent. La consommation d'aliments est suivie deux fois par semaine, et le nombre de décès deux fois par jour.

Les quatre types de régime alimentaire sont :
- régime 1, aliment synthétique appauvri en polyamines (PDD) et enrichi en huile d'olive (20 g / kg d'aliment),
- régime 2, aliment synthétique appauvri en polyamines (PDD) et enrichi en alkylglycérol 18 :1 (25 mg par jour et par souris),
- régime 3, aliment synthétique contenant des polyamines (PCD), enrichi en huile d'olive (20 g / kg d'aliment),
- régime 4, aliment synthétique contenant des polyamines (PCD), enrichi en alkylglycérol 18 :1 (25 mg par jour et par souris).

Les résultats de médiane de survie sont comparés deux à deux en fonction des régimes alimentaires.
Le régime 1 (durée médiane 23 jours) et le régime 2 (durée médiane 26 jours) ne montrent pas de différence significative concernant la durée médiane de survie (p = 0,50, Ratio 0,88, χ²=0,45 (ddl = 1)) (Figure 3).
Le régime 3 (durée médiane 24 jours) et le régime 4 (durée médiane 26 jours) ne montrent pas de différence significative concernant la durée médiane de survie (p = 0,86, Ratio 0,92, χ²=0,03 (ddl = 1)) (Figure 4).
Le régime 2 (durée médiane 26 jours) et le régime 3 (durée médiane 24 jours) ne montrent pas de différence significative concernant la durée médiane de survie (p = 0,39, Ratio 0,92, χ²=0,73 (ddl = 1)) (Figure 5).
Le régime 1 (durée médiane 23 jours) et le régime 3 (durée médiane 24 jours) ne montrent pas de différence significative concernant la durée médiane de survie (p = 0,83, Ratio 0,95, χ²=0,04 (ddl = 1)) (Figure 6).
Le régime 2 (durée médiane 26 jours) et le régime 4 (durée médiane 26 jours) ne montrent pas de différence significative concernant la durée médiane de survie (p = 0,37, Ratio 1, χ²=0,37 (ddl = 1)) (Figure 7).

L'utilisation d'un régime appauvri en polyamines, ou contenant des polyamines, et l'utilisation d'un régime contenant de l'alkylglycérol ou de l'huile d'olive n'a pas d'influence significative sur la durée médiane de survie.

### 3 Survie et Croissance tumorale

Le modèle animal (souris C57BL/5) et le modèle tumoral (carcinome de Lewis) sont décrits précédemment. Les souris sont greffées avec 0,1 × 10° cellules tumorales, par injection intramusculaire. Les souris sont divisées en cinq groupes et des régimes alimentaires différents sont administrés à chaque groupe.
Le premier groupe est alimenté avec un régime contenant un taux normal de polyamines (PCD), et enrichi en huile d'olive.
Le deuxième groupe est alimenté avec un régime contenant un taux normal de polyamines (PCD), et enrichi en alkylglycérol 18 :1 (alcool sélachylique).
Le troisième groupe est alimenté avec un régime appauvri en polyamines (PDD), et enrichi en alkylglycérol 18 :1.
Le quatrième groupe est alimenté avec un régime appauvri en polyamines (PDD), enrichi en huile d'olive, et enrichi en DFMO.
Le cinquième groupe est alimenté avec un régime appauvri en polyamines, enrichi en alkylglycérol 18 :1, et enrichi en DFMO.

L'huile d'olive est mélangée à l'alimentation des souris (20 g / kg d'aliment). L'alkylglycérol est le C 18 :1 (alcool sélachylique) et est mélangé à l'alimentation des souris (25 mg par jour et par souris). Le DFMO est mélangé à l'eau de boisson des souris (30 g/L).

L'évolution du volume des tumeurs (en mm³) est indiquée sur la figure 1. L'évolution de la survie des souris est indiquée sur la figure 2.

La croissance tumorale est plus lente pour le cinquième groupe de souris (groupe alimenté avec un régime appauvri en polyamines, enrichi en alkylglycérol et enrichi en DFMO) (Figure 1).

La survie est plus importante pour le cinquième groupe de souris (Figure 2).

Les souris du groupe alimentées avec un régime appauvri en polyamines, enrichi en alkylglycérol et enrichi en DFMO, ne présentent pas de métastase pulmonaire contrairement aux autres groupes. On constate une splenomégalie chez toutes les souris, cependant elle est moins importante pour les souris du cinquième groupe. Les souris du cinquième groupe sont en bonne forme et leur mort ne parait pas prévisible.

43 jours après la greffe de la tumeur toutes les souris sont mortes, à l'exception des souris du cinquième groupe. Cependant, elles sont moins vives et leurs poils sont hérissés.

### 4 Effet préventif et/ou curatif

Le modèle animal (souris C57BL/5) et le modèle tumoral (carcinome de Lewis) sont décrits précédemment. Les souris sont greffées avec 0,1 × 10° cellules tumorales, par injection intramusculaire.

Trois ensembles d'animaux sont constitués : A, B, C.

L'ensemble A commence le traitement sept jours avant la greffe de cellules tumorales. L'ensemble B commence le traitement le jour de la greffe de cellules tumorales. L'ensemble C commence le traitement sept jours après la greffe de cellules tumorales.

Chaque ensemble est subdivisé en 8 groupes distincts (soit 24 groupes au total), chaque groupe est alimenté avec un régime alimentaire différent.
Le groupe 1a : aliment synthétique contenant des polyamines (PCD), enrichi en huile d'olive,
le groupe 1b : aliment synthétique contenant des polyamines (PCD), enrichi en alkylglycérol,
le groupe 1c : aliment synthétique contenant des polyamines (PCD), et avec une eau contenant du DFMO,
le groupe 1d : aliment synthétique contenant des polyamines (PCD), en alkylglycérol, et avec une eau contenant du DFMO,
le groupe 2a : aliment synthétique appauvri en polyamines (PDD), enrichi en huile d'olive ,
le groupe 2b : aliment synthétique appauvri en polyamines (PDD), enrichi en alkylglycérol,
le groupe 2c : aliment synthétique appauvri en polyamines (PDD), et avec une eau contenant du DFMO,
le groupe 2d : aliment synthétique appauvri en polyamines (PDD), en alkylglycérol, et avec une eau contenant du DFMO.

L'huile d'olive est mélangée à l'alimentation des souris (20 g / kg d'aliment). L'alkylglycérol est le C 18 :1 (alcool sélachylique) et est mélangé à l'alimentation des souris (25 mg par jour et par souris). Le DFMO est mélangé à l'eau de boisson des souris (30 g/L).

Pour chacun des 8 groupes des 3 ensembles, la croissance de la tumeur est quantifiée tous les deux jours par mesure du volume de la tumeur, selon la méthode décrite précédemment (Moulinoux et al., Int J. Cancer, 1984, 34(2), 277-281).

La survie des animaux dans les différents groupes est mesurée.

### 5 Agent anti-tumoral

### Exemple 1

Le modèle animal (souris C57BL/5) et le modèle tumoral (carcinome de Lewis) sont décrits précédemment. Les souris sont greffées avec 0,1 ×10⁶ cellules tumorales, par injection intramusculaire. Les souris sont divisées en quatre groupes et des régimes alimentaires différents sont administrés à chaque groupe.
Les premier groupe et deuxième groupes sont alimentés avec un régime appauvri en polyamines (PDD), et enrichi en alkylglycérol 18 :1 (alcool sélachyique) (25 mg par jour et par souris).
Les troisième et quatrième groupes sont alimentés avec un régime appauvri en polyamines (PDD), et enrichi en huile d'olive (20 g / kg d'aliment).
Les deuxième et quatrième groupes sont également traités par injection de cyclophosphamide.

Le cyclophosphamide est administré par injection intra péritonéale (cyclophosphamide dans NaCl 0,9%, 20 mg/kg/semaine, 100 µL/souris).

Le traitement débute lorsque la tumeur est palpable.

L'évolution du volume des tumeurs (en mm³) est indiquée dans la figure 9. L'évolution de la survie des souris est indiquée dans la figure 10, et le pourcentage moyen de survie est indiqué dans la figure 11.

La croissance tumorale est plus lente pour le deuxième groupe de souris (groupe alimenté avec un régime enrichi en alkylglycérol et avec injection de cyclophosphamide (Figure 9). La survie est également plus importante pour le deuxième groupe de souris (Figure 10 et11).

### Exemple 2

Le modèle animal (souris C57BL/5) et le modèle tumoral (carcinome de Lewis) sont décrits précédemment. Les souris sont greffées avec 0,1 ×10⁶ cellules tumorales, par injection intramusculaire.

Quatres ensembles d'animaux sont constitués : A, B, C et D.
L'ensemble A est traité avec une monochimiothérapie a forte dose (cyclophosphamide, 90 mg / kg / semaine).
L'ensemble B est traité avec une monochimiothérapie a faible dose (cyclophosphamide, 20 mg / kg / semaine).
L'ensemble C est traité avec une polychimiothérapie combinant trois agents antitumoraux (methotrexate, cyclophosphamide, vindesine). Le premier jour de l'expérience l'ensemble C est traité avec le methotrexate (1,7 mg / kg). Le second jour de l'expérience l'ensemble C est traité avec le cyclophosphamide (20 mg / kg). Le troisième jour de l'expérience l'ensemble C est traité avec la vindesine (0,25 mg/kg).
L'ensemble D est traité avec une solution saline (NaCl, 0,14 M), il s'agit d'un groupe témoin.

Pour chacun des 4 ensembles, les souris sont réparties en 8 groupes (soit 32 groupes au total) en fonction de leurs alimentations :
Le groupe 1a : aliment synthétique contenant des polyamines (PCD),
le groupe 1b : aliment synthétique contenant des polyamines (PCD), et avec une eau contenant du DFMO,
le groupe 1c : aliment synthétique contenant des polyamines (PCD), enrichi en alkylglycérol,
le groupe 1d : aliment synthétique contenant des polyamines (PCD), enrichi en alkylglycérol, et avec une eau contenant du DFMO,
le groupe 2a : aliment synthétique appauvri en polyamines (PDD),
le groupe 2b : aliment synthétique appauvri en polyamines (PDD), et avec une eau contenant du DFMO,
le groupe 2c : aliment synthétique appauvri en polyamines (PDD), enrichi en alkylglycérol,
le groupe 2d : aliment synthétique appauvri en polyamines (PDD), enrichi en alkylglycérol, et avec une eau contenant du DFMO,

L'alkylglycérol est le C 18 :1 (alcool sélachylique) et est mélangé à l'alimentation des souris (25 mg par jour et par souris). Le DFMO est mélangé à l'eau de boisson des souris (30 g/L).

Les animaux ont accès à la nourriture et à l'eau à volonté.

* Evolution de la tumeur

10 animaux par groupe sont euthanasiés 3 semaines après la greffe des cellules tumorales.

Durant les 3 semaines de l'expérience, le volume de la tumeur est estimé tous les deux jours.

Après euthanasie, les métastases pulmonaires sont comptées.

### * Survie

La survie de 20 animaux par groupe est observée.

La chimiothérapie, tout particulièrement le cyclophosphamide administré à faible dose aux animaux PDD + DFMO se révèle efficace vis-à-vis de la progression de la greffe tumorale et de la dissémination métastatique pulmonaire. Il en va de même pour la survie des animaux. L'absorption de C18 :1 amplifie les effets bénéfiques de la chimiothérapie associée à la réduction des apports exogènes (PDD) et endogènes (DFMO) de polyamines.

### Exemple 3

Survie et Croissance tumorale en fonction d'une alimentation pauvre en polyamines et d'un mélange naturel d'alkylglycérols

Caractérisation du mélange naturel d'Alkylglycérols : Le mélange naturel d'alkylglycérols est obtenu suite à la purification d'huile de foie de requin et comprend les proportions d'Alkylglycérols suivantes comme précédemment décrit (Pédrono et al., 2004) :

| Alkylglycérols | Composition préférée | Composition restreinte | Composition large |
|---|---|---|---|
| 14 :0 | 0,7 à 2,6 % | 0,5 à 3,5% | 0 à 10 % |
| 16 :0 | 9,1 à 12,5 % | 7 à 15 % | 0 à 30 % |
| 16 :1n-7 | 11 à 12,5 % | 9 à 15 % | 0 à 30 % |
| 18 :0 | 0 à 4,4 % | 0 à 7 % | 0 à 15 % |
| 18 :1 n-9 | 54,4 à 68,1 % | 45 à 75 % | 20 à 100 % |
| 18 :1 n-7 | 4,8 à 6,2 | 2 à 8 % | 0 à 20 % |
| Autres | 4,8 à 8,9 % | 2 à 10 % | 0 à 22 % |

Le modèle animal (souris C57BL/5) et le modèle tumoral (carcinome de Lewis) sont décrits précédemment. Les souris sont greffées avec 0,1 ×10⁶ cellules tumorales, par injection intramusculaire. Les souris sont divisées en cinq groupes et des régimes alimentaires différents sont administrés à chaque groupe.
Le premier groupe est alimenté avec un régime contenant un taux normal de polyamines (PCD), et enrichi en huile d'olive.
Le deuxième groupe est alimenté avec un régime contenant un taux normal de polyamines (PCD), et enrichi en mélange naturel d'alkylglycérol.
Le troisième groupe est alimenté avec un régime appauvri en polyamines (PDD), et enrichi en mélange naturel d'alkylglycérol.
Le quatrième groupe est alimenté avec un régime appauvri en polyamines (PDD), enrichi en huile d'olive, et enrichi en DFMO.
Le cinquième groupe est alimenté avec un régime appauvri en polyamines, enrichi en mélange naturel d'alkylglycérol., et enrichi en DFMO.

L'huile d'olive est mélangée à l'alimentation des souris (20 g / kg d'aliment). Le mélange naturel d'alkylglycérols est mélangé à l'alimentation des souris (25 mg par jour et par souris). Le DFMO est mélangé à l'eau de boisson des souris (30 g/L).

La croissance tumorale est plus lente pour le cinquième groupe de souris (groupe alimenté avec un régime appauvri en polyamines, enrichi en alkylglycérols et enrichi en DFMO).

La survie est plus importante pour le cinquième groupe de souris.

Les souris du groupe alimentées avec un régime appauvri en polyamines, enrichi en alkylglycérols et enrichi en DFMO, ne présentent pas de métastase pulmonaire contrairement aux autres groupes. On constate une splénomégalie chez toutes les souris, cependant elle est moins importante pour les souris du cinquième groupe. Les souris du cinquième groupe sont en bonne forme et leur mort ne parait pas prévisible.

### Exemple 4

Survie et Croissance tumorale en fonction d'une alimentation pauvre en polyamines et d'un mélange naturel en alkylglycérols ou d'un alkylglycérol C18 :1 associé ou non à l'alpha-méthylornithine

Caractérisation du mélange naturel d'Alkylglycérols : Le mélange naturel d'alkylglycérols est obtenu suite à la purification d'huile de foie de requin et comprend les proportions d'Alkylglycérols suivantes comme précédemment décrit (Pedrono *et al.,* 2004) :
14 :0 : 0,7 à 2,6 %
16 :0 : 9,1 à 12,5 %
16 :ln-7: I1 à 12,5 %
18 :0 : 0 à 4,4 %
18 :1 n-9 : 54,4 à 68,1 %
18 :1 n-7 : 4,8 à 6,2 %
Autres : 4,8 à 8,9 %

Le modèle animal (souris C57BL/5) et le modèle tumoral (carcinome de Lewis) sont décrits précédemment. Les souris sont greffées avec 0,1 ×10⁶ cellules tumorales, par injection intramusculaire. Les souris sont divisées en cinq groupes et des régimes alimentaires différents sont administrés à chaque groupe.
Le premier groupe est alimenté avec un régime contenant un taux normal de polyamines (PCD), et enrichi en huile d'olive.
Le deuxième groupe est alimenté avec un régime contenant un taux normal de polyamines (PCD), et enrichi en mélange naturel d'alkylglycérols ou en alkylglycérol C18 :1.
Le troisième groupe est alimenté avec un régime appauvri en polyamines (PDD), et enrichi en mélange naturel d'alkylglycérols ou en alkylglycérol C18 :1.
Le quatrième groupe est alimenté avec un régime appauvri en polyamines (PDD), enrichi en huile d'olive, et enrichi en alpha-méthylornithine.
Le cinquième groupe est alimenté avec un régime appauvri en polyamines, enrichi en mélange naturel d'alkylglycérols ou en alkylglycérol C18 :1. et enrichi en alpha-méthylornithine.

L'huile d'olive est mélangée à l'alimentation des souris (20 g / kg d'aliment). Le mélange naturel d'alkylglycérols ou l'alkylglycérol C18 :1 est mélangé à l'alimentation des souris (25 mg par jour et par souris). L'alpha-méthylornithine est mélangée à l'eau de boisson des souris (30 g/L).

La croissance tumorale est plus lente pour le cinquième groupe de souris (groupe alimenté avec un régime appauvri en polyamines, enrichi en alkylglycérols et enrichi en alpha-méthylornithine).

La survie est plus importante pour le cinquième groupe de souris.

Les souris du groupe alimentées avec un régime appauvri en polyamines, enrichi en alkylglycérols et enrichi en alpha-méthylornithine, ne présentent pas de métastase pulmonaire contrairement aux autres groupes. On constate une splénomégalie chez toutes les souris, cependant elle est moins importante pour les souris du cinquième groupe. Les souris du cinquième groupe sont en bonne forme et leur mort ne parait pas prévisible.

### 6 Effet dose

Le modèle animal (souris C57BL/5) et le modèle tumoral (carcinome de Lewis) sont décrits précédemment. Les souris sont greffées avec 0,1 ×10⁶ cellules tumorales, par injection intramusculaire.

Les animaux sont divisés en 16 groupes, chaque groupe est alimenté avec un régime alimentaire différent.
Le groupe 1a : aliment synthétique appauvri en polyamines (PDD), enrichi en huile d'olive,
le groupe 1b : aliment synthétique appauvri en polyamines (PDD), enrichi en huile d'olive , et avec une eau contenant du DFMO,
le groupe 1c : aliment synthétique appauvri en polyamines (PDD), et avec une eau contenant du DFMO,
le groupe 1d : aliment synthétique appauvri en polyamines (PDD), enrichi en alkylglycérol (5 mg / jour), et avec une eau contenant du DFMO,
le groupe le : aliment synthétique appauvri en polyamines (PDD), enrichi en alkylglycérol (10 mg / jour), et avec une eau contenant du DFMO,
le groupe 1f: aliment synthétique appauvri en polyamines (PDD), enrichi en alkylglycérol (15 mg / jour), et avec eau contenant du DFMO,
le groupe 1g: aliment synthétique appauvri en polyamines (PDD), enrichi en alkylglycérol (20 mg / jour), et avec eau contenant du DFMO,
le groupe 1h: aliment synthétique appauvri en polyamines (PDD), enrichi en alkylglycérol (25 mg / jour), et avec une eau contenant du DFMO,
le groupe 2a : aliment synthétique contenant des polyamines (PCD), enrichi en huile d'olive,
le groupe 2b : aliment synthétique contenant des polyamines (PCD), enrichi en huile d'olive, et avec une eau contenant du DFMO,
le groupe 2c : aliment synthétique contenant des polyamines (PCD), et avec une eau contenant du DFMO,
le groupe 2d : aliment synthétique contenant des polyamines (PCD), enrichi en alkylglycérol (5 mg / jour), et avec une eau contenant du DFMO,
le groupe 2e : aliment synthétique contenant des polyamines (PCD), enrichi en alkylglycérol (10 mg / jour), et avec une eau contenant du DFMO,
le groupe 2f : aliment synthétique contenant des polyamines (PCD), enrichi en alkylglycérol (15 mg / jour), et avec une eau contenant du DFMO,
le groupe 2g : aliment synthétique contenant des polyamines (PCD), enrichi en alkylglycérol (20 mg / jour), et avec une eau contenant du DFMO,
le groupe 2h : aliment synthétique contenant des polyamines (PCD), enrichi en alkylglycérol (25 mg / jour), et avec une eau contenant du DFMO.

L'huile d'olive est mélangée à l'alimentation des souris (20 g / kg d'aliment). Le DFMO est mélangé à l'eau de boisson des souris (30 g/L).

Pour chacun des 16 groupes, la croissance de la tumeur est quantifiée tous les deux jours par mesure du volume de la tumeur, selon la méthode décrite précédemment (Moulinoux et al., Int J. Cancer, 1984, 34(2), 277-281).

La survie des animaux dans les différents groupes est mesurée.

### II Angiogénèse (référence)

### Matrigel

La néo vascularisation induite par le Matrigel est dosée suivant une modification de la méthode de Kimura et al. (Cancer Sci. 2004, 95(9), 758-764). En résumé, tous les animaux ont été traités par injection sous-cutanée de Matrigel (500µL) contenant 32 unités/mL d'héparine et éventuellement 50 ng/mL de VEGF (Vascular Endothelial Growth Factor).

### 1 Effet anti-angiogénique

Le modèle animal (souris C57BL/5) est décrit précédemment. Les souris sont greffées par injection intramusculaire du Matrigel au premier jour de l'expérience. Les traitements commencent une heure après greffe du Matrigel.

Les animaux sont divisés en 10 groupes, chaque groupe est alimenté avec un régime alimentaire différent.
Les groupes 1a et 1b : aliment synthétique contenant des polyamines (PCD), enrichi en huile d'olive,
le groupe 1c : aliment synthétique contenant des polyamines (PCD), enrichi en alkylglycérol,
le groupe 1d: aliment synthétique contenant des polyamines (PCD), et avec une eau contenant du DFMO,
le groupe le : aliment synthétique contenant des polyamines (PCD), enrichi en alkylglycérol (20 g / kg), et avec une eau contenant du DFMO.
Les groupes 2a et 2b : aliment synthétique appauvri en polyamines (PDD), enrichi en huile d'olive,
le groupe 2c : aliment synthétique appauvri en polyamines (PDD), enrichi en alkylglycérol,
le groupe 2d : aliment synthétique appauvri en polyamines (PDD), et avec une eau contenant du DFMO,
le groupe 2e : aliment synthétique appauvri en polyamines (PDD), enrichi en alkylglycérol, et avec une eau contenant du DFMO,

L'huile d'olive est mélangée à l'alimentation des souris (20 g / kg d'aliment). L'alkylglycérol est le C 18 :1 (alcool sélachylique) et est mélangé à l'alimentation des souris (25 mg par jour et par souris). Le DFMO est mélangé à l'eau de boisson des souris (30 g/L).

Les groupes 1a et 2a ont été greffés avec un Matrigel comprenant de l'héparine. Les groupes 1b, 1c, 1d, 1e, 2b, 2c, 2d et 2e ont été greffés avec un Matrigel comprenant de l'héparine et du VEGF.

Les animaux sont euthanasiés 10 jours après la greffe du Matrigel. Les gels sont récupérés et pesés, puis le contenu en hémoglobine des gels est déterminé en utilisant un kit hémoglobine (Wako Pure Chemical Co.)

### 2 Détection des effets anti angiogénique du C18 :1

Le modèle animal (souris C57BL/5) et le Matrigel sont décrits précédemment.

Le Matrigel est injecté en sous-cutané (500 µL par injection) à trois groupes de 6 souris :
Groupe 1 : Matrigel sans VEGF, régime alimentaire appauvri en polyamines (PDD).
Groupe 2 : Matrigel avec VEGF, régime alimentaire appauvri en polyamines (PDD).
Groupe 3 : Matrigel avec VEGF, régime alimentaire appauvri en polyamines (PDD), enrichi en alkylglycérol 18 :1 (25 mg par jour et par souris)

Dix jours après injection les souris sont euthanasiées et le Matrigel est prélevé, pesé, photographié. Le Matrigel est ensuite dissocié mécaniquement dans du PBS, homogénéisé puis est mis dans une solution de Drabkin's pour le dosage de l'hémoglobine. Les solutions sont déposées dans des puits d'une plaque 96 puits contenant une solution de Drabkin's et lecture dans un lecteur de plaque à 540 nm.

La concentration en hémoglobine observe dans le Matrigel du groupe 2 est fixé arbitrairement à 100% et sert de référence lors des comparaisons avec les résultats obtenus avec les groupes 1 et 3 (Figure 8).

Le groupe 1, sans VEGF (groupe témoin), a une concentration d'hémoglobine de 21,63% par rapport au groupe 2.

Le groupe 3 avec VEGF et traitement C 18 :1, a une concentration d'hémoglobine de 58,56% par rapport au groupe 2 (test de Mann et Whithney p=0,0086). Le C18 :1 a réduit d'environ 40% l'angiogénèse.

Un effet anti angiogénique de l'alkylglycérol est observé.

## Revendications

1. Utilisation d'un produit contenant :
- au moins un alkylglycérol,
- au moins un inhibiteur de la biosynthèse des polyamines, ledit inhibiteur étant un inhibiteur de l'ornithine décarboxylase,
- un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et
- au moins un agent antitumoral,
comme préparation de combinaison pour la fabrication d'un médicament destiné au traitement de pathologies tumorales,
la quantité totale de polyamines biologiquement «actives» par jour ingérées par l'homme ou l'animal, auquel le susdit médicament est administré, ne dépassant pas 30 nanomoles par kcal de produit ingéré, notamment 25 nanomoles par kcal de produit ingéré, notamment 23 nanomoles par kcal de produit ingéré, notamment 20 nanomoles par kcal de produit ingéré,
ladite utilisation étant une utilisation simultanée, séparée ou étalée dans le temps,

2. Utilisation selon la revendication 1, d'un produit contenant :
- une combinaison d'un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine,
et d'au moins un alkylglycérol,
- au moins un inhibiteur de la biosynthèse des polyamines, ledit inhibiteur étant un inhibiteur de l'ornithine décarboxylase,
et
- au moins un agent antitumoral,
comme préparation de combinaison pour la fabrication d'un médicament destiné au traitement de pathologies tumorales,
la quantité totale de polyamines biologiquement «actives» par jour ingérées par l'homme ou l'animal, auquel le susdit médicament est administré, ne dépassant pas 30 nano moles par kcal de produit ingéré, notamment 25 nanomoles par kcal de produit ingéré, notamment 23 nanomoles par kcal de produit ingéré, notamment 20 nanomoles par kcal de produit ingéré,
ladite utilisation étant une utilisation simultanée, séparée ou étalée dans le temps.

3. Utilisation selon l'une des revendications 1 ou 2, d'un produit contenant :
- une combinaison d'un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine,
d'au moins un alkylglycérol et d'au moins un inhibiteur de la biosynthèse des polyamines, ledit inhibiteur étant un inhibiteur de l'ornithine décarboxylase,
et
- au moins un agent antitumoral
comme préparation de combinaison pour la fabrication d'un médicament destiné au traitement de pathologies tumorales,
la quantité totale de polyamines biologiquement «actives» par jour ingérées par l'homme ou l'animal, auquel le susdit médicament est administré, ne dépassant pas 30 nano moles par kcal de produit ingéré, notamment 25 nanomoles par kcal de produit ingéré, notamment 23 nanomoles par kcal de produit ingéré, notamment 20 nanomoles par kcal de produit ingéré,
ladite utilisation étant une utilisation simultanée, séparée ou étalée dans le temps.

4. Utilisation selon l'une des revendications 1, 2 ou 3, d'une composition contenant au moins un alkylglycérol, au moins un inhibiteur de la biosynthèse des polyamines, un excipient appauvri en polyamines biologiquement «actives», et contenant au moins un agent antitumoral,
pour la préparation d'un médicament, pour l'homme ou l'animal, destiné au traitement de pathologies tumorales,
la quantité totale de polyamines biologiquement «actives» par jour ingérées par l'homme ou l'animal, auquel le susdit médicament est administré, ne dépassant pas 30 nano moles par kcal de produit ingéré, notamment 25 nanomoles par kcal de produit ingéré, notamment 23 nanomoles par kcal de produit ingéré, notamment 20 nanomoles par kcal de produit ingéré.

5. Utilisation selon l'une des revendications 1 à 4, d'un produit dans lequel l'alkylglycérol peut être énantiomériquement pur, racémique ou énantiomériquement enrichi,
ledit alkylglycérol étant un dérivé du glycérol dans lequel l'une au moins des fonctions hydroxyle est alkylé par une chaîne alkyle de 10 à 30 atomes de carbone, notamment de 10 à 20 atomes de carbone, notamment de 15 à 20 atomes de carbone, saturée ou non, substituée ou non.

6. Utilisation selon l'une des revendications 1 à 5, d'un produit pour la préparation d'un médicament, lequel médicament contient moins de 30 nanomoles de polyamines biologiquement «actives» par millilitre de médicament, notamment 14 nanomoles de polyamines biologiquement «actives» par millilitre de médicament, particulièrement moins de 2 nanomoles de polyamines biologiquement «actives» par millilitre de médicament, lesdites polyamines biologiquement actives étant choisies parmi la putrescine, la cadavérine, la spermine et la spermidine,

7. Utilisation selon l'une des revendications 1 à 6, d'un produit dans laquelle l'inhibiteur de la biosynthèse des polyamines correspondant à un inhibiteur de l'ornithine décarboxylase, est un dérivé de l'ornithine, particulièrement la 2-2'-(Difluorométhyl) ornithine (DFMO).

8. Produit contenant :
- au moins un alkylglycérol,
- au moins un inhibiteur de la biosynthèse des polyamines, ledit inhibiteur étant un inhibiteur de l'ornithine décarboxylase,
- un excipient appauvri en polyamines biologiquement «actives» correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et
- au moins un agent antitumoral,
comme préparation de combinaison pour une utilisation simultanée, séparée ou étalée dans le temps,
ladite préparation de combinaison comprenant au maximum 9 mg de polyamines biologiquement « actives » par unité journalière de composition.

9. Composition contenant :
- au moins un alkylglycérol,
- au moins un inhibiteur de la biosynthèse des polyamines, ledit inhibiteur étant un inhibiteur de l'ornithine décarboxylase,
- un excipient appauvri en polyamines biologiquement «actives», correspondant à un excipient dans lequel le taux total de polyamines actives est 100 fois inférieur, notamment 500 fois inférieur, notamment 1000 fois inférieur à la quantité de polyamines actives présentes dans un régime alimentaire normal contenant 40 à 360 nmol de polyamines biologiquement actives par kcal par jour, lesdites polyamines biologiquement actives étant choisies parmi la putrescine, la cadavérine, la spermine et la spermidine, et contenant
- au moins un agent antitumoral,
ladite composition comprenant au maximum 9 mg de polyamines biologiquement « actives » par unité journalière de composition.

10. Produit selon la revendication 8 contenant moins de 30 nanomoles de polyamines biologiquement «actives» par kcal de produit, notamment 14 nanomoles de polyamines biologiquement «actives» par kcal de produit, particulièrement moins de 2 nanomoles de polyamines biologiquement «actives» par kcal de produit.

11. Produit selon l'une des revendications 8 ou 10, comprenant au minimum 20 µg, notamment 10 µg, notamment 1 µg de polyamines biologiquement « actives » par unité journalière de produit.

12. Produit selon les revendications l'une des revendications 8, 10 ou 11, contenant :
- de 10 ng / kcal à 1 µg / kcal de l'alkylglycérol C18 :1,
- environ 9 mg de DFMO,
- un excipient contenant de 1 µg à 9 mg de polyamines biologiquement «actives», et
- de 100 mg à 200 mg de cyclophosphamide.

## Patentansprüche

1. Verwendung eines Produkts, enthaltend:
- mindestens ein Alkylglycerol,
- mindestens einen Inhibitor der Biosynthese von Polyaminen, wobei der Inhibitor ein Inhibitor von Ornithindecarboxylase ist,
- einen Exzipienten, der an biologisch "aktiven" Polyaminen verarmt ist, und der einem Exzipienten entspricht, in dem der Gesamtgehalt an aktiven Polyaminen 100-mal kleiner, insbesondere 500-mal kleiner, insbesondere 1000-mal kleiner ist als die Menge an aktiven Polyaminen, die in einem normalen Ernährungsplan vorhanden sind, der 40 bis 360 nmol biologisch aktive Polyamine pro kcal pro Tag enthält, wobei die biologisch aktiven Polyamine aus Putrescin, Cadaverin, Spermin und Spermidin ausgewählt sind, und
- mindestens ein Anti-Tumormittel,
als Kombinationszubereitung zur Herstellung eines Medikaments, das zur Behandlung von Tumorpathologien bestimmt ist,
wobei die Gesamtmenge an biologisch "aktiven" Polyaminen pro Tag, die von einem Menschen oder einem Tier zu sich genommen werden, dem das Medikament verabreicht wird, 30 Nanomol pro kcal des zu sich genommenen Produkts, insbesondere 25 Nanomol pro kcal des zu sich genommenen Produkts, insbesondere 23 Nanomol pro kcal des zu sich genommenen Produkts, insbesondere 20 Nanomol pro kcal des zu sich genommenen Produkts nicht überschreitet,
wobei die Verwendung eine gleichzeitige, getrennte oder zeitlich gestaffelte Verwendung ist.

2. Verwendung nach Anspruch 1 eines Produkts, enthaltend:
- eine Kombination eines Exzipienten, der an biologisch "aktiven" Polyaminen verarmt ist, und der einem Exzipienten entspricht, in dem der Gesamtgehalt an aktiven Polyaminen 100-mal kleiner, insbesondere 500-mal kleiner, insbesondere 1000-mal kleiner ist als die Menge an aktiven Polyaminen, die in einem normalen Ernährungsplan vorhanden sind, der 40 bis 360 nmol biologisch aktive Polyamine pro kcal pro Tag enthält, wobei die biologisch aktiven Polyamine aus Putrescin, Cadaverin, Spermin und Spermidin ausgewählt sind,
und mindestens eines Alkylglycerols,
- mindestens einen Inhibitor der Biosynthese von Polyaminen, wobei der Inhibitor ein Inhibitor von Ornithindecarboxylase ist, und
- mindestens ein Anti-Tumormittel,
als Kombinationszubereitung zur Herstellung eines Medikaments, das zur Behandlung von Tumorpathologien bestimmt ist,
wobei die Gesamtmenge an biologisch "aktiven" Polyaminen pro Tag, die von einem Menschen oder einem Tier zu sich genommen werden, dem das Medikament verabreicht wird, 30 Nanomol pro kcal des zu sich genommenen Produkts, insbesondere 25 Nanomol pro kcal des zu sich genommenen Produkts, insbesondere 23 Nanomol pro kcal des zu sich genommenen Produkts, insbesondere 20 Nanomol pro kcal des zu sich genommenen Produkts nicht überschreitet,
wobei die Verwendung eine gleichzeitige, getrennte oder zeitlich gestaffelte Verwendung ist.

3. Verwendung nach einem der Ansprüche 1 oder 2 eines Produkts, enthaltend:
- eine Kombination eines Exzipienten, der an biologisch "aktiven" Polyaminen verarmt ist, und der einem Exzipienten entspricht, in dem der Gesamtgehalt an aktiven Polyaminen 100-mal kleiner, insbesondere 500-mal kleiner, insbesondere 1000-mal kleiner ist als die Menge an aktiven Polyaminen, die in einem normalen Ernährungsplan vorhanden sind, der 40 bis 360 nmol biologisch aktive Polyamine pro kcal pro Tag enthält, wobei die biologisch aktiven Polyamine aus Putrescin, Cadaverin, Spermin und Spermidin ausgewählt sind,
mindestens eines Alkylglycerols und mindestens eines Inhibitors der Biosynthese von Polyaminen, wobei der Inhibitor ein Inhibitor von Ornithindecarboxylase ist,
und
- mindestens ein Anti-Tumormittel,
als Kombinationszubereitung zur Herstellung eines Medikaments, das zur Behandlung von Tumorpathologien bestimmt ist,
wobei die Gesamtmenge an biologisch "aktiven" Polyaminen pro Tag, die von einem Menschen oder einem Tier zu sich genommen werden, dem das Medikament verabreicht wird, 30 Nanomol pro kcal des zu sich genommenen Produkts, insbesondere 25 Nanomol pro kcal des zu sich genommenen Produkts, insbesondere 23 Nanomol pro kcal des zu sich genommenen Produkts, insbesondere 20 Nanomol pro kcal des zu sich genommenen Produkts nicht überschreitet,
wobei die Verwendung eine gleichzeitige, getrennte oder zeitlich gestaffelte Verwendung ist.

4. Verwendung nach einem der Ansprüche 1, 2 oder 3 einer Zusammensetzung, enthaltend mindestens ein Alkylglycerol, mindestens einen Inhibitor der Biosynthese von Polyaminen, einen Exzipienten, der an biologisch "aktiven" Polyaminen verarmt ist, und enthaltend mindestens ein Anti-Tumormittel,
zur Herstellung eines Medikaments, für Menschen oder Tiere, das zur Behandlung von Tumorpathologien bestimmt ist,
wobei die Gesamtmenge an biologisch "aktiven" Polyaminen pro Tag, die von einem Menschen oder einem Tier zu sich genommen werden, dem das Medikament verabreicht wird, 30 Nanomol pro kcal des zu sich genommenen Produkts, insbesondere 25 Nanomol pro kcal des zu sich genommenen Produkts, insbesondere 23 Nanomol pro kcal des zu sich genommenen Produkts, insbesondere 20 Nanomol pro kcal des zu sich genommenen Produkts nicht überschreitet.

5. Verwendung nach einem der Ansprüche 1 bis 4 eines Produkts, in dem Alkylglycerol enantiomer rein, racemisch oder enantiomer angereichert sein kann,
wobei das Alkylglycerol ein Derivat von Glycerol ist, in dem mindestens eine der Hydroxylfunktionen durch eine Alkylkette mit 10 bis 30 Kohlenstoffatomen, insbesondere von 10 bis 20 Kohlenstoffatomen, insbesondere von 15 bis 20 Kohlenstoffatomen, gesättigt oder nicht, substituiert oder nicht, alkyliert ist.

6. Verwendung nach einem der Ansprüche 1 bis 5 eines Produkts zur Herstellung eines Medikaments, wobei das Medikament weniger als 30 Nanomol biologisch "aktive" Polyamine pro Milliliter des Medikaments enthält, insbesondere 14 Nanomol biologisch "aktive" Polyamine pro Milliliter des Medikaments, vorzugsweise weniger als 2 Nanomol biologisch "aktive" Polyamine pro Milliliter des Medikaments, wobei die biologisch aktiven Polyamine ausgewählt sind aus Putrescin, Cadaverin, Spermin und Spermidin.

7. Verwendung nach einem der Ansprüche 1 bis 6 eines Produkts, wobei der Inhibitor der Biosynthese von Polyaminen, der einem Inhibitor von Ornithindecarboxylase entspricht, ein Derivat von Orniothin ist, insbesondere 2-2'-(Difluormethyl)-ornithin (DFMO).

8. Produkt, enthaltend:
- mindestens ein Alkylglycerol,
- mindestens einen Inhibitor der Biosynthese von Polyaminen, wobei der Inhibitor ein Inhibitor von Ornithindecarboxylase ist,
- einen Exzipienten, der an biologisch "aktiven" Polyaminen verarmt ist, und der einem Exzipienten entspricht, in dem der Gesamtgehalt an aktiven Polyaminen 100-mal kleiner, insbesondere 500-mal kleiner, insbesondere 1000-mal kleiner ist als die Menge an aktiven Polyaminen, die in einem normalen Ernährungsplan vorhanden sind, der 40 bis 360 nmol biologisch aktive Polyamine pro kcal pro Tag enthält, wobei die biologisch aktiven Polyamine aus Putrescin, Cadaverin, Spermin und Spermidin ausgewählt sind, und
- mindestens ein Anti-Tumormittel,
als Kombinationszubereitung für eine gleichzeitige, getrennte oder zeitlich gestaffelte Verwendung,
wobei die Kombinationszubereitung maximal 9 mg biologisch "aktive" Polyamine pro Tageseinheit der Zusammensetzung umfasst.

9. Zusammensetzung, enthaltend:
- mindestens ein Alkylglycerol,
- mindestens einen Inhibitor der Biosynthese von Polyaminen, wobei der Inhibitor ein Inhibitor von Ornithindecarboxylase ist,
- einen Exzipienten, der an biologisch "aktiven" Polyaminen verarmt ist, und der einem Exzipienten entspricht, in dem der Gesamtgehalt an aktiven Polyaminen 100-mal kleiner, insbesondere 500-mal kleiner, insbesondere 1000-mal kleiner ist als die Menge an aktiven Polyaminen, die in einem normalen Ernährungsplan vorhanden sind, der 40 bis 360 nmol biologisch aktive Polyamine pro kcal pro Tag enthält, wobei die biologisch aktiven Polyamine aus Putrescin, Cadaverin, Spermin und Spermidin ausgewählt sind, und enthaltend
- mindestens ein Anti-Tumormittel,
wobei die Zusammensetzung maximal 9 mg biologisch "aktive" Polyamine pro Tageseinheit der Zusammensetzung umfasst.

10. Produkt nach Anspruch 8, enthaltend weniger als 30 Nanomol biologisch "aktive" Polyamine pro kcal des Produkts, insbesondere 14 Nanomol biologisch "aktive" Polyamine pro kcal des Produkts, vorzugsweise weniger als 2 Nanomol biologisch "aktive" Polyamine pro kcal des Produkts.

11. Produkt nach einem der Ansprüche 8 oder 10, enthaltend mindestens 20 µg, insbesondere 10 µg, insbesondere 1 µg biologisch "aktive" Polyamine pro Tageseinheit des Produkts.

12. Produkt nach einem der Ansprüche 8, 10 oder 11, enthaltend:
- von 10 ng / kcal bis 1 µg / kcal Alkylglycerol C18:1,
- ungefähr 9 mg DMFO,
- einen Exzipienten, enthaltend 1 µg bis 9 mg biologisch "aktive" Polyamine,
- von 100 mg bis 200 mg Cyclophosphamid.

## Claims

1. Use of a product containing:
- at least one alkylglycerol,
- at least one inhibitor of the bio-synthesis of polyamines, said inhibitor being an inhibitor of ornithine decarboxylase,
- an excipient depleted of biologically "active" polyamines, corresponding to an excipient in which the total level of active polyamines is 100 times lower, in particular 500 times lower, in particular 1000 times lower than the amount of active polyamines present in a normal diet containing 40 to 360 nmol of biologically "active" polyamines per kcal per day, said biologically "active" polyamines being chosen from putrescine, cadaverine, spermine and spermidine, and
- at least one anti-tumor agent,
as a combination preparation for the manufacture of a medicament for the treatment of tumor pathologies,
the total amount of biologically "active" polyamines per day ingested by humans or animals, to which the above-mentioned medicinal product is administered, not exceeding 30 nano moles per kcal of ingested product, in particular 25 nanomoles per kcal of ingested product, in particular 23 nanomoles per kcal of ingested product, in particular 20 nanomoles by kcal of ingested product,
said use being for simultaneous, separate or spread over time use.

2. Use according to claim 1 of a product containing:
- a combination of an excipient depleted of biologically "active" polyamines, corresponding to an excipient in which the total level of active polyamines is 100 times lower, in particular 500 times lower, in particular 1000 times lower than the amount of active polyamines present in a normal diet containing 40 to 360 nmol of biologically "active" polyamines per kcal per day, said biologically "active" polyamines being chosen from putrescine, cadaverine, spermine and spermidine, and of at least one alkylglycerol,
- at least one inhibitor of the bio-synthesis of polyamines, said inhibitor being an inhibitor of ornithine decarboxylase,
and
- at least one anti-tumor agent,
as a combination preparation for the manufacture of a medicament for the treatment of tumor pathologies,
the total amount of biologically "active" polyamines per day ingested by humans or animals, to which the above-mentioned medicinal product is administered, not exceeding 30 nano moles per kcal of ingested product, in particular 25 nanomoles per kcal of ingested product, in particular 23 nanomoles per kcal of ingested product, in particular 20 nanomoles by kcal of ingested product,
said use being for simultaneous, separate or spread over time use.

3. Use according to one of claims 1 or 2, of a product containing:
- a combination of an excipient depleted of biologically "active" polyamines, corresponding to an excipient in which the total level of active polyamines is 100 times lower, in particular 500 times lower, in particular 1000 times lower than the amount of active polyamines present in a normal diet containing 40 to 360 nmol of biologically "active" polyamines per kcal per day, said biologically "active" polyamines being chosen from putrescine, cadaverine, spermine and spermidine,
of at least one alkylglycerol and of at least one inhibitor of the bio-synthesis of polyamines, said inhibitor being an inhibitor of ornithine decarboxylase,
and
- at least one anti-tumor agent
as a combination preparation for the manufacture of a medicament for the treatment of tumor pathologies,
the total amount of biologically "active" polyamines per day ingested by humans or animals, to which the above-mentioned medicinal product is administered, not exceeding 30 nano moles per kcal of ingested product, in particular 25 nanomoles per kcal of ingested product, in particular 23 nanomoles per kcal of ingested product, in particular 20 nanomoles by kcal of ingested product,
said use being for simultaneous use, separate or spread over time.

4. Use according to one of claims 1, 2 or 3 of a composition containing at least one alkylglycerol, at least one one inhibitor of the bio-synthesis of polyamines, an excipient depleted of biologically "active" polyamines, and containing at least one anti-tumor agent,
for the manufacture of a medicament, for humans or animals, for the treatment of tumor pathologies,
the total amount of biologically "active" polyamines per day ingested by humans or animals, to which the above-mentioned medicinal product is administered, not exceeding 30 nano moles per kcal of ingested product, in particular 25 nanomoles per kcal of ingested product, in particular 23 nanomoles per kcal of ingested product, in particular 20 nanomoles by kcal of ingested product.

5. Use according to one of claims 1 to 4 of a product in which the alkylglycerol can be enantiomerically pure, racemic or enantiomerically enriched,
said alkylglycerol being a derivative of glycerol in which at least one of the hydroxyl functions is alkylated by an alkyl chain of 10 to 30 carbon atoms, especially 10 to 20 carbon atoms, especially 15 to 20 carbon atoms, saturated or not, substituted or not.

6. Use according to one of claims 1 to 5 of a product for the preparation of a medicament, which medicament contains less than 30 nanomoles of biologically "active" polyamines per milliliter of medicament, in particular 14 nanomoles of biologically "active" polyamines per milliliter of medicament, particularly less than 2 nanomoles of biologically "active" polyamines per milliliter of medicament, said biologically "active" polyamines being chosen from putrescine, cadaverine, spermine and spermidine.

7. Use according to one of claims 1 to 6 of a product in which the inhibitor of the bio-synthesis of polyamines, corresponding to an inhibitor of ornithine decarboxylase, is a derivative of ornithine, particularly the 2-2'-(Difluoromethyl) ornithine (DFMO).

8. Product containing:
- at least one alkylglycerol,
- at least one inhibitor of the bio-synthesis of polyamines, said inhibitor being an inhibitor of ornithine decarboxylase,
- an excipient depleted of biologically "active" polyamines, corresponding to an excipient in which the total level of active polyamines is 100 times lower, in particular 500 times lower, in particular 1000 times lower than the amount of active polyamines present in a normal diet containing 40 to 360 nmol of biologically "active" polyamines per kcal per day, said biologically "active" polyamines being chosen from putrescine, cadaverine, spermine and spermidine, and
- at least one anti-tumor agent,
as a combination preparation for simultaneous, separate or spread over time use, said combination preparation comprising at most 9 mg of biologically "active" polyamines per daily unit of composition.

9. Composition containing:
- at least one alkylglycerol,
- at least one inhibitor of the bio-synthesis of polyamines, said inhibitor being an inhibitor of ornithine decarboxylase,
- an excipient depleted of biologically "active" polyamines, corresponding to an excipient in which the total level of active polyamines is 100 times lower, in particular 500 times lower, in particular 1000 times lower than the amount of active polyamines present in a normal diet containing 40 to 360 nmol of biologically "active" polyamines per kcal per day, said biologically "active" polyamines being chosen from putrescine, cadaverine, spermine and spermidine,
and containing
- at least one anti-tumor agent,
said composition comprising at most 9 mg of biologically "active" polyamines per daily unit of composition.

10. Product according claim 8 containing less than 30 nanomoles of biologically "active" polyamines per kcal of product, in particular 14 nanomoles of biologically "active" polyamines per kcal of product, particularly less than 2 nanomoles of biologically "active" polyamines per kcal of product.

11. Product according to one of claims 8 or 10 comprising at least 20 µg, in particular 10 µg, in particular 1 µg of biologically "active" polyamines per daily product unit.

12. Product according to one of claims 8, 10 or 11 containing:
- from 10 ng / kcal to 1 µg / kcal of C18:1 alkylglycerol,
- about 9 mg of DFMO,
- an excipient containing from 1 µg to 9 mg biologically "active" polyamines, and
- from 100 mg to 200 mg of cyclophosphamide.
